# FASCICULE DE BREVET EUROPEEN

(11) **EP 1 705 188 B1**
(45) Date de publication et mention de la délivrance du brevet: **31.12.2014**
(21) Numéro de dépôt: 06290416.4
(22) Date de dépôt: 14.03.2006
(51) Int. Cl.: C07K 14/47, A61K 38/17, C07K 1/113, C07K 1/107, A61K 8/64, A61Q 19/00

(54) **Polypeptides modifiés de la famille des KAP et utilisation en cosmétique**
Modifizierte Polypeptide aus der KAP-Familie und deren kosmetische Verwendung
Modified KAP familiy polypeptides and their use in cosmetics

(30) Priorité: 18.03.2005 FR 0550713
(43) Date de publication de la demande: 27.09.2006
(73) Titulaire: L'Oréal, 75008 Paris (FR)
(72) Inventeur: Philippe, Michel, 91320 Wissous (FR)
(74) Mandataire: Allab, Myriam

(56) Documents cités:
- WO-A-99/49837
- DATABASE WPI Section Ch, Week 200346 Derwent Publications Ltd., London, GB; Class B04, AN 2003-493307 XP002355971 & WO 03/042387 A1 (JAPAN SOC PROMOTION SCI) 22 mai 2003 (2003-05-22)
- LIU S M ET AL: "Transsulfuration, protein synthesis rate and follicle mRNA in the skin of young merino lambs in response to infusions of methionine and serine" BRITISH JOURNAL OF NUTRITION, CAMBRIDGE, GB, vol. 83, 2000, pages 401-409, XP002986973
- TOBIN DESMOND J ET AL: "The lysosomal protease cathepsin L is an important regulator of keratinocyte and melanocyte differentiation during hair follicle morphogenesis and cycling", AMERICAN JOURNAL OF PATHOLOGY, vol. 160, no. 5, May 2002 (2002-05), pages 1807-1821, ISSN: 0002-9440
- O'GUIN W M ET AL: "INTERACTION OF TRICHOHYALIN WITH INTERMEDIATE FILAMENTS THREE IMMUNOLOGICALLY DEFINED STAGES OF TRICHOHYALIN MATURATION", JOURNAL OF INVESTIGATIVE DERMATOLOGY, vol. 98, no. 1, 1992, pages 24-32, ISSN: 0022-202X
- TARCSA EDIT ET AL: "The fate of trichohyalin: Sequential post-translational modifications by peptidyl-arginine deiminase and transglutaminases", JOURNAL OF BIOLOGICAL CHEMISTRY, vol. 272, no. 44, 31 October 1997 (1997-10-31), pages 27893-27901, ISSN: 0021-9258
- SHIMOMURA YUTAKA ET AL: "Human hair keratin-associated proteins", JOURNAL OF INVESTIGATIVE DERMATOLOGY SYMPOSIUM PROCEEDINGS, vol. 10, no. 3, December 2005 (2005-12), pages 230-233, ISSN: 1087-0024
- THIBAUT SEBASTIEN ET AL: "Transglutaminase-3 Enzyme: A Putative Actor in Human Hair Shaft Scaffolding?", JOURNAL OF INVESTIGATIVE DERMATOLOGY, vol. 129, no. 2, February 2009 (2009-02), pages 449-459, ISSN: 0022-202X

### SEQUENCE LISTING

<110> L'OREAL
<120> POLYNUCLEOTIDES MODIFIES DE LA FAMILLE DES KAP ET UTILISATION EN COSMETIQUE
<130> 0A05077/CB
<140> 06290416.4 <141> 2006-03-14
<160> 66
<170> PatentIn version 3.3
<210> 1
   <211> 177
   <212> PRT
   <213> Homo sapiens
<400> 1
   Met Ala Cys Cys Gln Thr Ser Phe Cys Gly Phe Pro Ser Cys Ser Thr
   1               5                   10                  15
   Ser Gly Thr Cys Gly Ser Ser Cys Cys Gln Pro Ser Cys Cys Glu Thr
               20                  25                  30
   Ser Ser Cys Gln Pro Arg Cys Cys Glu Thr Ser Cys Cys Gln Pro Ser
           35                  40                  45
   Cys Cys Gln Thr Ser Phe Cys Gly Phe Pro Ser Phe Ser Thr Gly Gly
       50                  55                  60
   Thr Cys Asp Ser Ser Cys Cys Gln Pro Ser Cys Cys Glu Thr Ser Cys
   65                  70                  75                  80
   Cys Gln Pro Ser Cys Tyr Gln Thr Ser Ser Cys Gly Thr Gly Cys Gly
                   85                  90                  95
   Ile Gly Gly Gly Ile Gly Tyr Gly Gln Glu Gly Ser Ser Gly Ala Val
               100                 105                 110
   Ser Thr Arg Ile Arg Trp Cys Arg Pro Asp Cys Arg Val Glu Gly Thr
           115                 120                 125
   Cys Leu Pro Pro Cys Cys Val Val Ser Cys Thr Pro Pro Ser Cys Cys
       130                 135                 140
   Gln Leu His His Ala Glu Ala Ser Cys Cys Arg Pro Ser Tyr Cys Gly
   145                 150                 155                 160
   Gln Ser Cys Cys Arg Pro Val Cys Cys Cys Tyr Cys Ser Glu Pro Thr
                   165                 170                 175
   Cys
<210> 2
   <211> 167
   <212> PRT
   <213> Homo sapiens
<400> 2
   Met Thr Cys Cys Gln Thr Ser Phe Cys Gly Tyr Pro Ser Cys Ser Thr
   1               5                   10                  15
   Ser Gly Thr Cys Gly Ser Ser Cys Cys Gln Pro Ser Cys Cys Glu Thr
               20                  25                  30
   Ser Cys Cys Gln Pro Ser Cys Cys Gln Thr Ser Phe Cys Gly Phe Pro
           35                  40                  45
   Ser Phe Ser Thr Ser Gly Thr Cys Ser Ser Ser Cys Cys Gln Pro Ser
       50                  55                  60
   Cys Cys Glu Thr Ser Cys Cys Gln Pro Ser Cys Cys Gln Thr Ser Ser
   65                  70                  75                  80
   Cys Gly Thr Gly Cys Gly Ile Gly Gly Gly Ile Gly Tyr Gly Gln Glu
                   85                  90                  95
   Gly Ser Ser Gly Ala Val Ser Thr Arg Ile Arg Trp Cys Arg Pro Asp
               100                 105                 110
   Cys Arg Val Glu Gly Thr Cys Leu Pro Pro Cys Cys Val Val Ser Cys
           115                 120                 125
   Thr Pro Pro Thr Cys Cys Gln Leu His His Ala Glu Ala Ser Cys Cys
       130                 135                 140
   Arg Pro Ser Tyr Cys Gly Gln Ser Cys Cys Arg Pro Val Cys Cys Cys
   145                 150                 155                 160
   Tyr Ser Cys Glu Pro Thr Cys
                   165
<210> 3
   <211> 174
   <212> PRT
   <213> Homo sapiens
<400> 3
   Met Thr Cys Cys Gln Thr Ser Phe Cys Gly Tyr Pro Ser Phe Ser Ile
   1               5                   10                  15
   Ser Gly Thr Cys Gly Ser Ser Cys Cys Gln Pro Ser Cys Cys Glu Thr
               20                  25                  30
   Ser Cys Cys Gln Pro Arg Ser Cys Gln Thr Ser Phe Cys Gly Phe Pro
           35                  40                  45
   Ser Phe Ser Thr Ser Gly Thr Cys Ser Ser Ser Cys Cys Gln Pro Ser
       50                  55                  60
   Cys Cys Glu Thr Ser Cys Cys Gln Pro Ser Cys Cys Glu Thr Ser Cys
   65                  70                  75                  80
   Cys Gln Pro Ser Cys Cys Gln Ile Ser Ser Cys Gly Thr Gly Cys Gly
                   85                  90                  95
   Ile Gly Gly Gly Ile Ser Tyr Gly Gln Glu Gly Ser Ser Gly Ala Val
               100                 105                 110
   Ser Thr Arg Ile Arg Trp Cys Arg Pro Asp Ser Arg Val Glu Gly Thr
           115                 120                 125
   Tyr Leu Pro Pro Cys Cys Val Val Ser Cys Thr Pro Pro Ser Cys Cys
       130                 135                 140
   Gln Leu His His Ala Gln Ala Ser Cys Cys Arg Pro Ser Tyr Cys Gly
   145                 150                 155                 160
   Gln Ser Cys Cys Arg Pro Val Cys Cys Cys Glu Pro Thr Cys
                   165                 170
<210> 4
   <211> 131
   <212> PRT
   <213> Homo sapiens
<400> 4
   Met Ala Cys Cys Gln Thr Ser Phe Cys Gly Phe Arg Ser Phe Ser Thr
   1               5                   10                  15
   Gly Gly Thr Cys Asp Ser Ser Cys Cys Gln Pro Ser Cys Cys Glu Thr
               20                  25                  30
   Ser Cys Cys Gln Pro Ser Cys Tyr Gln Thr Ser Ser Cys Gly Thr Gly
           35                  40                  45
   Cys Gly Ile Gly Gly Gly Ile Gly Tyr Gly Gln Glu Gly Ser Ser Gly
       50                  55                  60
   Ala Val Ser Thr Arg Ile Arg Trp Cys Arg Pro Asp Cys Arg Val Glu
   65                  70                  75                  80
   Gly Thr Cys Leu Pro Pro Cys Cys Val Val Ser Cys Thr Pro Pro Ser
                   85                  90                  95
   Cys Cys Gln Leu His His Ala Glu Ala Ser Cys Cys Arg Pro Ser Tyr
               100                 105                 110
   Cys Gly Gln Ser Cys Cys Arg Pro Val Cys Cys Cys Tyr Cys Ser Glu
           115                 120                 125
   Pro Thr Cys
       130
<210> 5
   <211> 85
   <212> PRT
   <213> Homo sapiens
<400> 5
   Met Ala Cys Cys Gln Thr Ser Phe Cys Gly Phe Pro Ser Cys Ser Thr
   1               5                   10                  15
   Ser Gly Ala Val Ser Thr Arg Ile Arg Trp Cys Arg Pro Asp Cys Arg
               20                  25                  30
   Val Glu Gly Thr Cys Leu Pro Pro Cys Cys Val Val Ser Cys Thr Pro
           35                  40                  45
   Pro Ser Cys Cys Gln Leu His His Ala Glu Ala Ser Cys Cys Arg Pro
       50                  55                  60
   Ser Tyr Cys Gly Gln Ser Cys Cys Arg Pro Val Cys Cys Cys Tyr Cys
   65                  70                  75                  80
   Ser Glu Pro Thr Cys
                   85
<210> 6
   <211> 177
   <212> PRT
   <213> Homo sapiens
<400> 6
<210> 7
   <211> 141
   <212> PRT
   <213> Homo sapiens
<400> 7
   Met Thr Cys Cys Gln Thr Ser Phe Cys Gly Tyr Pro Ser Cys Ser Thr
   1               5                   10                  15
   Ser Gly Thr Cys Gly Ser Ser Cys Cys Gln Pro Ser Cys Cys Glu Thr
               20                  25                  30
   Ser Cys Cys Gln Pro Ser Cys Cys Glu Thr Ser Cys Cys Gln Pro Ser
           35                  40                  45
   Cys Cys Gln Thr Ser Ser Cys Gly Thr Gly Cys Gly Ile Gly Gly Gly
       50                  55                  60
   Ile Gly Tyr Gly Gln Glu Gly Ser Ser Gly Ala Val Ser Thr Arg Ile
   65                  70                  75                  80
   Arg Trp Cys Arg Pro Asp Cys Arg Val Glu Gly Thr Cys Leu Pro Pro
                   85                  90                  95
   Cys Cys Val Val Ser Cys Thr Pro Pro Thr Cys Cys Gln Leu His His
               100                 105                 110
   Ala Glu Ala Ser Cys Cys Arg Pro Ser Tyr Cys Gly Gln Ser Cys Cys
           115                 120                 125
   Arg Pro Val Cys Cys Cys Tyr Ser Cys Glu Pro Thr Cys
       130                 135                 140
<210> 8
   <211> 96
   <212> PRT
   <213> Homo sapiens
<400> 8
   Pro Val Thr Cys Gln Thr Thr Val Cys Arg Pro Val Thr Cys Val Pro
   1               5                   10                  15
   Arg Cys Thr Arg Pro Ile Cys Glu Pro Cys Arg Arg Pro Val Cys Cys
               20                  25                  30
   Asp Pro Cys Ser Leu Gln Glu Gly Cys Cys Arg Pro Ile Thr Cys Cys
           35                  40                  45
   Pro Ser Ser Cys Thr Ala Val Val Cys Arg Pro Cys Cys Trp Ala Thr
       50                  55                  60
   Thr Cys Cys Gln Pro Val Ser Val Gln Ser Pro Cys Cys Arg Pro Pro
   65                  70                  75                  80
   Cys Gly Gln Pro Thr Pro Cys Ser Thr Thr Cys Arg Thr Ser Ser Cys
                   85                  90                  95
<210> 9
   <211> 33
   <212> PRT
   <213> Homo sapiens
<400> 9
   Thr Thr Cys Cys Gln Pro Val Ser Val Gln Ser Pro Cys Cys Arg Pro
   1               5                   10                  15
   Pro Cys Gly Gln Pro Thr Pro Cys Ser Thr Thr Cys Arg Thr Ser Ser
               20                  25                  30
   Cys
<210> 10
   <211> 85
   <212> PRT
   <213> Homo sapiens
<400> 10
   Val Thr Cys Val Pro Arg Cys Thr Arg Pro Ile Cys Glu Pro Cys Arg
   1               5                   10                  15
   Arg Pro Val Cys Cys Asp Pro Cys Ser Leu Gln Glu Gly Cys Cys Arg
               20                  25                  30
   Pro Ile Thr Cys Cys Pro Ser Ser Cys Thr Ala Val Val Cys Arg Pro
           35                  40                  45
   Cys Cys Trp Ala Thr Thr Cys Cys Gln Pro Val Ser Val Gln Ser Pro
       50                  55                  60
   Cys Cys Arg Pro Pro Cys Gly Gln Pro Thr Pro Cys Ser Thr Thr Cys
   65                  70                  75                  80
   Arg Thr Ser Ser Cys
                   85
<210> 11
   <211> 128
   <212> PRT
   <213> Homo sapiens
<400> 11
   Met Thr Gly Ser Cys Cys Gly Ser Thr Leu Ser Ser Leu Ser Tyr Gly
   1               5                   10                  15
   Gly Gly Cys Cys Gln Pro Cys Cys Cys Arg Asp Pro Cys Cys Cys Arg
               20                  25                  30
   Pro Val Thr Cys Gln Thr Thr Val Cys Arg Pro Val Thr Cys Val Pro
           35                  40                  45
   Arg Cys Thr Arg Pro Ile Cys Glu Pro Cys Arg Arg Pro Val Cys Cys
       50                  55                  60
   Asp Pro Cys Ser Leu Gln Glu Gly Cys Cys Arg Pro Ile Thr Cys Cys
   65                  70                  75                  80
   Pro Ser Ser Cys Thr Ala Val Val Cys Arg Pro Cys Cys Trp Ala Thr
                   85                  90                  95
   Thr Cys Cys Gln Pro Val Ser Val Gln Ser Pro Cys Cys Arg Pro Pro
               100                 105                 110
   Cys Gly Gln Pro Thr Pro Cys Ser Thr Thr Cys Arg Thr Ser Ser Cys
           115                 120                 125
<210> 12
   <211> 98
   <212> PRT
   <213> Homo sapiens
<400> 12
   Met Tyr Cys Cys Ala Leu Arg Ser Cys Ser Val Pro Thr Gly Pro Ala
   1               5                   10                  15
   Thr Thr Phe Cys Ser Phe Asp Lys Ser Cys Arg Cys Gly Val Cys Leu
               20                  25                  30
   Pro Ser Thr Cys Pro His Glu Ile Ser Leu Leu Gln Pro Ile Cys Cys
           35                  40                  45
   Asp Thr Cys Pro Pro Pro Cys Cys Lys Pro Asp Thr Tyr Val Pro Thr
       50                  55                  60
   Cys Trp Leu Leu Asn Asn Cys His Pro Thr Pro Gly Leu Ser Gly Ile
   65                  70                  75                  80
   Asn Leu Thr Thr Tyr Val Gln Pro Gly Cys Glu Ser Pro Cys Glu Pro
                   85                  90                  95
   Arg Cys
<210> 13
   <211> 98
   <212> PRT
   <213> Homo sapiens
<400> 13
   Met Asp Cys Cys Ala Ser Arg Ser Cys Ser Val Pro Thr Gly Pro Ala
   1               5                   10                  15
   Thr Thr Ile Cys Ser Ser Asp Lys Ser Cys Arg Cys Gly Val Cys Leu
               20                  25                  30
   Pro Ser Thr Cys Pro His Thr Val Trp Leu Leu Glu Pro Ile Cys Cys
           35                  40                  45
   Asp Asn Cys Pro Pro Pro Cys His Ile Pro Gln Pro Cys Val Pro Thr
       50                  55                  60
   Cys Phe Leu Leu Asn Ser Cys Gln Pro Thr Pro Gly Leu Glu Thr Leu
   65                  70                  75                  80
   Asn Leu Thr Thr Phe Thr Gln Pro Cys Cys Glu Pro Cys Leu Pro Arg
                   85                  90                  95
   Gly Cys
<210> 14
   <211> 98
   <212> PRT
   <213> Homo sapiens
<400> 14
   Met Asp Cys Cys Ala Ser Arg Gly Cys Ser Val Pro Thr Gly Pro Ala
   1               5                   10                  15
   Thr Thr Ile Cys Ser Ser Asp Lys Ser Cys Arg Cys Gly Val Cys Leu
   20 25 30
   Pro Ser Thr Cys Pro His Thr Val Trp Leu Leu Glu Pro Thr Cys Cys
   35 40 45
   Asp Asn Cys Pro Pro Pro Cys His Ile Pro Gln Pro Cys Val Pro Thr
   50 55 60
   Cys Phe Leu Leu Asn Ser Cys Gln Pro Thr Pro Gly Leu Glu Thr Leu
   65 70 75 80
   Asn Leu Thr Thr Phe Thr Gln Pro Cys Cys Glu Pro Cys Leu Pro Arg
   85 90 95
   Gly Cys
<210> 15
   <211> 136
   <212> PRT
   <213> Homo sapiens
<400> 15
   Met Val Asn Ser Cys Cys Gly Ser Val Cys Ser Asp Gln Gly Cys Gly
   1 5 10 15
   Leu Glu Asn Cys Cys Arg Pro Ser Cys Cys Gln Thr Thr Cys Cys Arg
   20 25 30
   Thr Thr Cys Cys Arg Pro Ser Cys Cys Val Ser Ser Cys Cys Arg Pro
   35 40 45
   Gln Cys Cys Gln Ser Val Cys Cys Gln Pro Thr Cys Cys Ser Pro Ser
   50 55 60
   Cys Cys Gln Thr Thr Cys Cys Arg Thr Thr Cys Cys Arg Pro Ser Cys
   65 70 75 80
   Cys Val Ser Ser Cys Phe Arg Pro Gln Cys Cys Gln Ser Val Cys Cys
   85 90 95
   Gln Pro Thr Cys Cys Arg Pro Ser Cys Gly Gln Thr Thr Cys Cys Arg
   100 105 110
   Thr Thr Cys Tyr Arg Pro Ser Cys Cys Val Ser Thr Cys Cys Arg Pro
   115 120 125
   Thr Cys Ser Ser Gly Ser Cys Cys
   130 135
<210> 16
   <211> 98
   <212> PRT
   <213> Homo sapiens
<400> 16
   Thr Cys Cys Arg Pro Ser Cys Cys Ile Ser Ser Cys Cys Arg Pro Ser
   1               5                   10                  15
   Cys Cys Ile Ser Ser Cys Cys Lys Pro Ser Cys Cys Gln Thr Thr Cys
               20                  25                  30
   Cys Arg Pro Ser Cys Cys Ile Ser Ser Cys Tyr Arg Pro Gln Cys Cys
           35                  40                  45
   Gln Pro Ser Cys Cys Arg Pro Ala Cys Cys Ile Ser Ser Cys Cys His
       50                  55                  60
   Pro Ser Cys Cys Val Ser Ser Cys Arg Cys Pro Phe Ser Cys Pro Thr
   65                  70                  75                  80
   Thr Cys Cys Arg Thr Thr Cys Phe His Pro Ile Cys Cys Gly Ser Ser
                   85                  90                  95
   Cys Cys
<210> 17
   <211> 166
   <212> PRT
   <213> Homo sapiens
<400> 17
   Met Val Asn Ser Cys Cys Gly Ser Val Cys Ser Asp Gln Gly Cys Gly
   1 5 10 15
   Leu Glu Asn Cys Cys Arg Pro Ser Tyr Cys Gln Thr Thr Cys Cys Arg
   20 25 30
   Thr Thr Cys Cys Arg Pro Ser Cys Cys Val Ser Ser Cys Cys Arg Pro
   35 40 45
   Gln Cys Cys Gln Thr Thr Cys Cys Arg Thr Thr Cys Cys His Pro Ser
   50 55 60
   Cys Cys Val Ser Ser Cys Cys Arg Pro Gln Cys Cys Gln Ser Val Cys
   65 70 75 80
   Cys Gln Pro Thr Cys Cys Arg Pro Gln Cys Cys Gln Thr Thr Cys Cys
   85 90 95
   Arg Thr Thr Cys Cys Arg Pro Ser Cys Cys Arg Pro Gln Cys Cys Gln
   100 105 110
   Ser Val Cys Cys Gln Pro Thr Cys Cys Cys Pro Ser Tyr Cys Val Ser
   115 120 125
   Ser Cys Cys Arg Pro Gln Cys Cys Gln Thr Thr Cys Cys Arg Thr Thr
   130 135 140
   Cys Cys Arg Pro Ser Cys Cys Val Ser Arg Cys Tyr Arg Pro His Cys
   145 150 155 160
   Gly Gln Ser Leu Cys Cys
   165
<210> 18
   <211> 186
   <212> PRT
   <213> Homo sapiens
<400> 18
   Met Val Ser Ser Cys Cys Gly Ser Val Ser Ser Glu Gln Ser Cys Gly
   1               5                   10                  15
   Leu Glu Asn Cys Cys Cys Pro Ser Cys Cys Gln Thr Thr Cys Cys Arg
               20                  25                  30
   Thr Thr Cys Cys Arg Pro Ser Cys Cys Lys Pro Gln Cys Cys Gln Ser
           35                  40                  45
   Val Cys Tyr Gln Pro Thr Cys Cys His Pro Ser Cys Cys Ile Ser Ser
       50                  55                  60
   Cys Cys Arg Pro Tyr Cys Cys Glu Ser Ser Cys Cys Arg Pro Cys Cys
   65                  70                  75                  80
   Cys Arg Pro Ser Cys Cys Gln Thr Thr Cys Cys Arg Thr Thr Cys Cys
                   85                  90                  95
   Arg Thr Thr Cys Cys Cys Pro Ser Cys Cys Val Ser Ser Cys Cys Arg
               100                 105                 110
   Pro Gln Cys Cys Gln Ser Val Cys Cys Gln Pro Thr Cys Cys Arg Pro
           115                 120                 125
   Ser Tyr Cys Ile Ser Ser Cys Cys His Pro Ser Cys Cys Glu Ser Ser
       130                 135                 140
   Cys Cys Arg Pro Cys Cys Cys Val Arg Pro Val Cys Gly Arg Val Ser
   145                 150                 155                 160
   Cys His Thr Thr Cys Tyr Arg Pro Thr Cys Val Ile Ser Thr Cys Pro
                   165                 170                 175
   Arg Pro Leu Cys Cys Ala Ser Ser Cys Cys
               180                 185
<210> 19
   <211> 109
   <212> PRT
   <213> Homo sapiens
<400> 19
   Ser Pro Leu Ala Ala Val Pro Ala Ala Ala Ser Pro Ala Ala Val Ala
   1               5                   10                  15
   Pro Ala Ala Val Cys Pro Gly Ala Ala Gly Pro Ser Ala Ala Ser Leu
               20                  25                  30
   Cys Ala Ala Ser Gln Pro Ala Ala Val Pro Ala Ala Ala Ser Pro Ala
           35                  40                  45
   Ala Ala Ala Pro Leu Ala Val Asn Pro Ala Ala Ala Ala Arg Ala Ala
       50                  55                  60
   Ala Ala Pro Ala Ala Ala Cys Val Gln Ser Val Ala Glu Ser Pro Ala
   65                  70                  75                  80
   Thr Pro Leu Ala Ile Ala Gln Pro Val Ser Phe Pro Pro Ala Pro Val
                   85                  90                  95
   Pro Cys Ala Val Pro Pro Leu Ala Ala Glu Leu Leu Ala
               100                 105
<210> 20
   <211> 210
   <212> PRT
   <213> Homo sapiens
<400> 20
   Met Val Ser Ser Cys Cys Gly Ser Val Cys Ser Asp Gln Gly Cys Gly
   1               5                   10                  15
   Gln Val Leu Cys Gln Glu Thr Cys Cys Arg Pro Ser Cys Cys Gln Thr
               20                  25                  30
   Thr Cys Cys Arg Thr Thr Cys Tyr Arg Pro Ser Cys Cys Val Ser Ser
           35                  40                  45
   Cys Cys Arg Pro Gln Cys Cys Gln Ser Val Cys Cys Gln Pro Thr Cys
       50                  55                  60
   Cys Arg Pro Ser Cys Cys Glu Thr Thr Cys Cys His Pro Arg Cys Cys
   65                  70                  75                  80
   Ile Ser Ser Cys Cys Arg Pro Ser Cys Cys Met Ser Ser Cys Cys Lys
                   85                  90                  95
   Pro Gln Cys Cys Gln Ser Val Cys Cys Gln Pro Thr Cys Cys His Pro
               100                 105                 110
   Ser Cys Cys Ile Ser Ser Cys Cys Arg Pro Ser Cys Cys Val Ser Arg
           115                 120                 125
   Cys Cys Arg Pro Gln Cys Cys Gln Ser Val Cys Cys Gln Pro Thr Cys
       130                 135                 140
   Cys Arg Pro Ser Cys Cys Ile Ser Ser Cys Cys Arg Pro Ser Cys Cys
   145                 150                 155                 160
   Glu Ser Ser Cys Cys Arg Pro Cys Cys Cys Arg Pro Cys Cys Cys Leu
                   165                 170                 175
   Arg Pro Val Cys Gly Arg Val Ser Cys His Thr Thr Cys Tyr Arg Pro
               180                 185                 190
   Thr Cys Val Ile Ser Thr Cys Pro Arg Pro Leu Cys Cys Ala Ser Ser
           195                 200                 205
   Cys Cys
       210
<210> 21
   <211> 114
   <212> PRT
   <213> Homo sapiens
<400> 21
   Ser Ser Cys Cys Lys Pro Gln Cys Cys Gln Ser Val Cys Cys Gln Pro
   1 5 10 15
   Thr Cys Cys His Pro Ser Cys Cys Ile Ser Ser Cys Cys Arg Pro Ser
   20 25 30
   Cys Cys Val Ser Ser Cys Cys Lys Pro Gln Cys Cys Gln Ser Val Cys
   35 40 45
   Cys Gln Pro Thr Cys Cys His Pro Ser Cys Ser Ile Ser Ser Cys Cys
   50 55 60
   Arg Pro Ser Cys Cys Glu Ser Ser Cys Cys Arg Pro Cys Cys Cys Val
   65 70 75 80
   Arg Pro Val Cys Gly Arg Val Ser Cys His Thr Thr Cys Tyr Arg Pro
   85 90 95
   Thr Cys Val Ile Ser Thr Cys Pro Arg Pro Val Cys Cys Ala Ser Ser
   100 105 110
   Cys Cys
<210> 22
   <211> 191
   <212> PRT
   <213> Homo sapiens
<400> 22
   Cys Gln Glu Thr Cys Cys Arg Pro Ser Cys Cys Glu Thr Thr Cys Cys
   1               5                   10                  15
   Arg Thr Thr Cys Cys Arg Pro Ser Cys Cys Val Ser Ser Cys Cys Arg
               20                  25                  30
   Pro Gln Cys Cys Gln Ser Val Cys Cys Gln Pro Thr Cys Ser Arg Pro
           35                  40                  45
   Ser Cys Cys Gln Thr Thr Cys Cys Arg Thr Thr Cys Tyr Arg Pro Ser
       50                  55                  60
   Cys Cys Val Ser Ser Cys Cys Arg Pro Gln Cys Cys Gln Pro Val Cys
   65                  70                  75                  80
   Cys Gln Pro Thr Cys Cys Arg Pro Ser Cys Cys Glu Thr Thr Cys Cys
                   85                  90                  95
   His Pro Arg Cys Cys Ile Ser Ser Cys Cys Arg Pro Ser Cys Cys Val
               100                 105                 110
   Ser Ser Cys Cys Lys Pro Gln Cys Cys Gln Ser Val Cys Cys Gln Pro
           115                 120                 125
   Asn Cys Cys Arg Pro Ser Cys Ser Ile Ser Ser Cys Cys Arg Pro Ser
       130                 135                 140
   Cys Cys Glu Ser Ser Cys Cys Arg Pro Cys Cys Cys Val Arg Pro Val
   145                 150                 155                 160
   Cys Gly Arg Val Ser Cys His Thr Thr Cys Tyr Arg Pro Thr Cys Val
                   165                 170                 175
   Ile Ser Ser Cys Pro Arg Pro Leu Cys Cys Ala Ser Ser Cys Cys
               180                 185                 190
<210> 23
   <211> 127
   <212> PRT
   <213> Homo sapiens
<400> 23
   Met Val Asn Ser Cys Cys Gly Ser Val Cys Ser Asp Gln Gly Cys Asp
   1 5 10 15
   Gln Gly Leu Cys Gln Glu Thr Cys Cys Arg Pro Ser Cys Cys Gln Thr
   20 25 30
   Thr Cys Cys Cys Pro Ser Cys Val Val Ser Ser Cys Cys Arg Pro Ser
   35 40 45
   Cys Ser Gln Thr Thr Cys Cys Gln Thr Thr Cys Cys Arg Pro Ser Cys
   50 55 60
   Cys Arg Pro Val Cys Cys Gln Thr Thr Cys Arg Pro Ser Cys Gly Val
   65 70 75 80
   Ser Ser Cys Cys Arg Pro Leu Cys Cys Gln Thr Thr Cys Arg Pro Ser
   85 90 95
   Cys Gly Val Ser Ser Cys Cys Arg Pro Leu Cys Cys Gln Thr Thr Cys
   100 105 110
   Cys Arg Thr Thr Cys Cys Arg Pro Ser Cys Cys Gly Ser Ser Cys
   115 120 125
<210> 24
   <211> 201
   <212> PRT
   <213> Homo sapiens
<400> 24
   Met Val Asn Ser Cys Cys Gly Ser Val Cys Ser Asp Gln Gly Cys Gly
   1 5 10 15
   Leu Glu Asn Cys Cys Arg Pro Ser Cys Cys Gln Thr Thr Cys Cys Arg
   20 25 30
   Thr Thr Cys Cys Arg Pro Ser Cys Cys Val Ser Ser Cys Cys Arg Pro
   35 40 45
   Gln Cys Cys Gln Ser Val Cys Cys Gln Pro Thr Cys Cys Arg Pro Ser
   50 55 60
   Cys Cys Gln Thr Thr Cys Cys Arg Thr Thr Cys Cys Arg Pro Ser Cys
   65 70 75 80
   Cys Val Ser Ser Cys Cys Arg Pro Gln Cys Cys Gln Ser Val Cys Cys
   85 90 95
   Gln Pro Thr Cys Cys Arg Pro Ser Cys Cys Gln Thr Thr Cys Cys Arg
   100 105 110
   Thr Thr Cys Cys Arg Pro Ser Cys Cys Val Ser Ser Cys Cys Arg Pro
   115 120 125
   Gln Cys Cys Gln Ser Val Cys Cys Gln Pro Thr Cys Cys Arg Pro Ser
   130 135 140
   Cys Cys Ile Ser Ser Ser Cys Cys Pro Ser Cys Cys Glu Ser Ser Cys
   145 150 155 160
   Cys Arg Pro Cys Cys Cys Leu Arg Pro Val Cys Gly Arg Val Ser Cys
   165 170 175
   His Thr Thr Cys Tyr Arg Pro Thr Cys Val Ile Ser Thr Cys Pro Arg
   180 185 190
   Pro Leu Cys Cys Ala Ser Ser Cys Cys
   195 200
<210> 25
   <211> 106
   <212> PRT
   <213> Homo sapiens
<400> 25
   Met Val Asn Ser Cys Cys Gly Ser Val Cys Ser Asp Gln Gly Cys Gly
   1 5 10 15
   Leu Glu Asn Cys Cys Arg Pro Ser Tyr Cys Gln Thr Thr Cys Cys Arg
   20 25 30
   Thr Thr Cys Cys Arg Pro Ser Cys Cys Arg Pro Ser Cys Cys Arg Pro
   35 40 45
   Gln Cys Cys Gln Ser Val Cys Cys Gln Pro Thr Cys Cys Cys Pro Ser
   50 55 60
   Tyr Cys Val Ser Ser Cys Cys Arg Pro Gln Cys Cys Gln Thr Thr Arg
   65 70 75 80
   Cys Arg Thr Thr Cys Cys Arg Pro Ser Cys Cys Val Ser Arg Cys Tyr
   85 90 95
   Arg Pro His Cys Gly Gln Ser Leu Cys Cys
   100 105
<210> 26
   <211> 195
   <212> PRT
   <213> Homo sapiens
<400> 26
   Met Val Asn Ser Cys Cys Gly Ser Val Cys Ser His Gln Gly Cys Gly
   1               5                   10                  15
   Gln Asp Leu Cys Gln Glu Thr Cys Cys Arg Pro Ser Cys Cys Glu Thr
               20                  25                  30
   Thr Cys Cys Arg Thr Thr Tyr Cys Arg Pro Ser Cys Cys Val Ser Ser
           35                  40                  45
   Cys Cys Arg Pro Gln Cys Cys Gln Ser Val Cys Cys Gln Pro Thr Cys
       50                  55                  60
   Cys Arg Pro Arg Cys Cys Ile Ser Ser Cys Cys Arg Pro Ser Cys Cys
   65                  70                  75                  80
   Val Ser Ser Cys Cys Lys Pro Gln Cys Cys Gln Ser Met Cys Cys Gln
                   85                  90                  95
   Pro Thr Cys Cys Arg Pro Arg Cys Cys Ile Ser Ser Cys Cys Arg Pro
               100                 105                 110
   Ser Cys Cys Val Ser Ser Cys Cys Arg Pro Gln Cys Cys Gln Ser Val
           115                 120                 125
   Cys Cys Gln Pro Thr Cys Cys His Pro Ser Cys Ser Ile Ser Ser Cys
       130                 135                 140
   Cys Arg Pro Ser Cys Cys Glu Ser Ser Cys Cys Arg Pro Cys Cys Cys
   145                 150                 155                 160
   Leu Arg Pro Val Cys Gly Gly Val Ser Cys His Thr Thr Cys Tyr Arg
                   165                 170                 175
   Pro Thr Cys Val Ile Ser Ser Cys Pro Arg Pro Leu Cys Cys Ala Ser
               180                 185                 190
   Ser Cys Cys
           195
<210> 27
   <211> 193
   <212> PRT
   <213> Homo sapiens
<400> 27
   Gln Gly Cys Gly Leu Glu Thr Cys Cys Arg Pro Ser Cys Cys Gln Thr
   1               5                   10                  15
   Thr Cys Cys Arg Thr Thr Cys Cys Arg Pro Ser Cys Cys Val Ser Ser
               20                  25                  30
   Cys Cys Arg Pro Gln Cys Cys Gln Ser Val Cys Cys Gln Pro Thr Cys
           35                  40                  45
   Cys Arg Pro Ser Cys Cys Pro Ser Cys Cys Gln Thr Thr Cys Cys Arg
       50                  55                  60
   Thr Thr Cys Cys Arg Pro Ser Cys Cys Val Ser Ser Cys Cys Arg Pro
   65                  70                  75                  80
   Gln Cys Cys Gln Ser Val Cys Cys Gln Pro Thr Cys Cys Arg Pro Ser
                   85                  90                  95
   Cys Ser Ile Ser Ser Cys Cys Arg Pro Ser Cys Cys Val Ser Arg Cys
               100                 105                 110
   Cys Arg Ser Gln Cys Cys Gln Ser Val Cys Cys Gln Pro Thr Cys Cys
           115                 120                 125
   Arg Pro Ser Cys Cys Ile Ser Ser Cys Cys Arg Pro Ser Cys Cys Glu
       130                 135                 140
   Ser Ser Cys Cys Arg Pro Cys Cys Cys Arg Pro Cys Cys Cys Leu Arg
   145                 150                 155                 160
   Pro Val Cys Gly Arg Val Ser Cys His Thr Thr Cys Tyr Arg Pro Thr
                   165                 170                 175
   Cys Val Ile Ser Thr Cys Pro Arg Pro Leu Cys Cys Ala Ser Ser Cys
               180                 185                 190
   Cys
<210> 28
   <211> 278
   <212> PRT
   <213> Homo sapiens
<400> 28
   Met Gly Cys Cys Gly Cys Ser Gly Gly Cys Gly Ser Ser Cys Gly Gly
   1               5                   10                  15
   Cys Gly Ser Gly Cys Gly Gly Cys Gly Ser Gly Cys Gly Gly Cys Gly
               20                  25                  30
   Ser Gly Cys Gly Gly Ser Gly Ser Ser Cys Cys Val Pro Val Cys Cys
           35                  40                  45
   Cys Lys Pro Val Cys Cys Arg Val Pro Thr Cys Ser Cys Ser Ser Cys
       50                  55                  60
   Gly Lys Gly Gly Cys Gly Ser Ser Gly Gly Ser Lys Gly Gly Cys Gly
   65                  70                  75                  80
   Ser Cys Gly Gly Cys Lys Gly Gly Cys Gly Ser Cys Gly Gly Ser Lys
                   85                  90                  95
   Gly Gly Cys Gly Ser Cys Gly Gly Ser Lys Gly Gly Cys Gly Ser Cys
               100                 105                 110
   Gly Gly Ser Lys Gly Gly Cys Gly Ser Gly Cys Gly Gly Cys Gly Ser
           115                 120                 125
   Ser Cys Cys Val Pro Val Cys Cys Cys Lys Pro Met Cys Cys Cys Val
       130                 135                 140
   Pro Ala Cys Ser Cys Ser Ser Cys Gly Lys Gly Gly Cys Gly Ser Cys
   145                 150                 155                 160
   Gly Cys Ser Lys Gly Ala Cys Gly Ser Cys Gly Gly Ser Lys Gly Gly
                   165                 170                 175
   Cys Gly Ser Cys Gly Gly Cys Lys Gly Gly Cys Gly Ser Cys Gly Gly
               180                 185                 190
   Ser Lys Gly Gly Cys Gly Ser Gly Cys Gly Gly Cys Gly Ser Gly Cys
           195                 200                 205
   Gly Val Pro Val Cys Cys Cys Ser Cys Ser Ser Cys Gly Ser Cys Ala
       210                 215                 220
   Gly Ser Lys Gly Gly Cys Gly Ser Ser Cys Ser Gln Cys Ser Cys Cys
   225                 230                 235                 240
   Lys Pro Cys Cys Cys Ser Ser Gly Cys Gly Ser Ser Cys Cys Gln Ser
                   245                 250                 255
   Ser Cys Cys Lys Pro Cys Cys Ser Gln Ser Ser Cys Cys Val Pro Val
               260                 265                 270
   Cys Cys Gln Cys Lys Ile
           275
<210> 29
   <211> 177
   <212> PRT
   <213> Homo sapiens
<400> 29
   Met Gly Cys Cys Gly Cys Ser Arg Gly Cys Gly Ser Gly Cys Gly Gly
   1 5 10 15
   Cys Gly Ser Ser Cys Gly Gly Cys Gly Ser Gly Cys Gly Gly Cys Gly
   20 25 30
   Ser Gly Arg Gly Gly Cys Gly Ser Gly Cys Gly Gly Cys Ser Ser Ser
   35 40 45
   Cys Gly Gly Cys Gly Ser Arg Cys Tyr Val Pro Val Cys Cys Cys Lys
   50 55 60
   Pro Val Cys Ser Trp Val Pro Ala Cys Ser Cys Thr Ser Cys Gly Ser
   65 70 75 80
   Cys Gly Gly Ser Lys Gly Gly Cys Gly Ser Cys Gly Gly Ser Lys Gly
   85 90 95
   Gly Cys Gly Ser Cys Gly Gly Ser Lys Gly Gly Cys Gly Ser Cys Gly
   100 105 110
   Cys Ser Gln Ser Ser Cys Cys Lys Pro Cys Cys Cys Ser Ser Gly Cys
   115 120 125
   Gly Ser Ser Cys Cys Gln Ser Ser Cys Cys Lys Pro Cys Cys Cys Gln
   130 135 140
   Ser Ser Cys Cys Val Pro Val Cys Cys Gln Ser Ser Cys Cys Lys Pro
   145 150 155 160
   Cys Cys Cys Gln Ser Asn Cys Cys Val Pro Val Cys Cys Gln Cys Lys
   165 170 175
   Ile
<210> 30
   <211> 238
   <212> PRT
   <213> Homo sapiens
<400> 30
   Met Gly Cys Ser Gly Cys Ser Gly Gly Cys Gly Ser Ser Cys Gly Gly
   1               5                   10                  15
   Cys Gly Ser Ser Cys Gly Gly Cys Gly Ser Gly Tyr Gly Gly Cys Gly
               20                  25                  30
   Ser Gly Cys Cys Val Pro Val Cys Cys Cys Lys Pro Val Cys Cys Cys
           35                  40                  45
   Val Pro Ala Cys Ser Cys Ser Ser Cys Gly Ser Cys Gly Gly Ser Lys
       50                  55                  60
   Gly Val Cys Gly Ser Cys Gly Gly Cys Lys Gly Gly Cys Gly Ser Cys
   65                  70                  75                  80
   Gly Gly Ser Lys Gly Gly Cys Gly Ser Ser Cys Cys Val Pro Val Cys
                   85                  90                  95
   Cys Ser Ser Ser Cys Gly Ser Cys Gly Gly Ser Lys Gly Val Cys Gly
               100                 105                 110
   Phe Arg Gly Gly Ser Lys Gly Gly Cys Gly Ser Cys Gly Cys Ser Gln
           115                 120                 125
   Cys Ser Cys Tyr Lys Pro Cys Cys Cys Ser Ser Gly Cys Gly Ser Ser
       130                 135                 140
   Cys Cys Gln Ser Ser Cys Cys Lys Pro Ser Cys Ser Gln Ser Ser Cys
   145                 150                 155                 160
   Cys Lys Pro Cys Cys Ser Gln Ser Ser Cys Cys Lys Pro Cys Cys Cys
                   165                 170                 175
   Ser Ser Gly Cys Gly Ser Ser Cys Cys Gln Ser Ser Cys Cys Lys Pro
               180                 185                 190
   Cys Cys Ser Gln Ser Ser Cys Cys Lys Pro Cys Cys Cys Ser Ser Gly
           195                 200                 205
   Cys Gly Ser Ser Cys Cys Gln Ser Ser Cys Cys Lys Pro Cys Ser Ser
       210                 215                 220
   Gln Ser Ser Cys Cys Val Pro Ile Cys Cys Gln Cys Lys Ile
   225                 230                 235
<210> 31
   <211> 288
   <212> PRT
   <213> Homo sapiens
<400> 31
   Met Gly Cys Cys Gly Cys Ser Gly Gly Cys Gly Ser Gly Cys Gly Gly
   1               5                   10                  15
   Cys Gly Ser Gly Cys Gly Gly Cys Gly Ser Gly Cys Gly Gly Cys Gly
               20                  25                  30
   Ser Gly Cys Gly Gly Cys Gly Ser Gly Cys Gly Gly Cys Gly Ser Ser
           35                  40                  45
   Cys Cys Val Pro Ile Cys Cys Cys Lys Pro Val Cys Cys Cys Val Pro
       50                  55                  60
   Ala Cys Ser Cys Ser Ser Cys Gly Ser Cys Gly Gly Ser Lys Gly Gly
   65                  70                  75                  80
   Tyr Gly Ser Cys Gly Gly Ser Lys Gly Gly Cys Val Ser Cys Gly Gly
                   85                  90                  95
   Ser Lys Gly Gly Cys Gly Ser Cys Gly Gly Ser Lys Gly Gly Cys Gly
               100                 105                 110
   Ser Cys Gly Gly Ser Lys Gly Gly Cys Gly Ser Cys Gly Gly Ser Lys
           115                 120                 125
   Gly Gly Cys Val Ser Cys Gly Gly Ser Lys Gly Gly Cys Gly Ser Cys
       130                 135                 140
   Gly Gly Ser Lys Gly Gly Cys Val Ser Cys Gly Gly Ser Lys Gly Gly
   145                 150                 155                 160
   Cys Gly Ser Cys Gly Gly Ser Lys Gly Gly Cys Gly Ser Cys Gly Gly
                   165                 170                 175
   Ser Lys Gly Gly Cys Gly Ser Cys Gly Gly Ser Lys Gly Gly Cys Gly
               180                 185                 190
   Ser Cys Gly Cys Ser Gln Cys Ser Cys Cys Lys Pro Cys Cys Cys Ser
           195                 200                 205
   Ser Gly Cys Gly Ser Ser Cys Cys Gln Ser Ser Cys Cys Lys Pro Cys
       210                 215                 220
   Cys Ser Ser Ser Gly Cys Gly Ser Ser Cys Cys Gln Ser Ser Cys Cys
   225                 230                 235                 240
   Lys Pro Tyr Cys Cys Gln Ser Ser Cys Cys Lys Pro Cys Cys Ser Ser
                   245                 250                 255
   Ser Gly Cys Gly Ser Ser Cys Cys Gln Ser Ser Cys Cys Asn Pro Cys
               260                 265                 270
   Cys Ser Gln Ser Ser Cys Cys Val Pro Val Cys Cys Gln Cys Lys Ile
           275                 280                 285
<210> 32
   <211> 221
   <212> PRT
   <213> Homo sapiens
<400> 32
   Met Gly Cys Cys Gly Cys Ser Gly Gly Cys Gly Ser Gly Cys Gly Gly
   1               5                   10                  15
   Leu Gly Ser Gly Cys Gly Gly Cys Gly Ser Gly Cys Gly Gly Arg Gly
               20                  25                  30
   Ser Gly Cys Gly Gly Cys Gly Ser Gly Cys Gly Gly Cys Gly Ser Gly
           35                  40                  45
   Cys Gly Gly Cys Gly Ser Gly Cys Gly Gly Cys Gly Gly Cys Gly Ser
   50 55 60
   Gly Cys Cys Val Pro Val Cys Cys Cys Lys Pro Met Cys Cys Cys Val
   65 70 75 80
   Pro Ala Cys Ser Cys Ser Ser Cys Gly Lys Gly Gly Cys Gly Ser Cys
   85 90 95
   Gly Gly Ser Lys Arg Gly Cys Val Ser Cys Gly Val Ser Lys Gly Ala
   100 105 110
   Cys Gly Ser Cys Gly Gly Ser Lys Gly Gly Cys Gly Ser Cys Gly Gly
   115 120 125
   Ser Lys Gly Gly Cys Gly Ser Cys Gly Gly Ser Lys Gly Gly Cys Gly
   130 135 140
   Ser Tyr Gly Cys Ser Gln Cys Ser Cys Cys Lys Pro Cys Cys Cys Ser
   145 150 155 160
   Ser Gly Cys Gly Ser Ser Cys Cys Gln Ser Ser Cys Cys Lys Pro Tyr
   165 170 175
   Cys Cys Gln Ser Ser Cys Cys Lys Pro Cys Ser Cys Phe Ser Gly Cys
   180 185 190
   Gly Ser Ser Cys Cys Gln Ser Ser Cys Tyr Lys Pro Cys Cys Cys Gln
   195 200 205
   Ser Ser Cys Cys Val Pro Val Cys Cys Gln Cys Lys Ile
   210 215 220
<210> 33
   <211> 129
   <212> PRT
   <213> Homo sapiens
<400> 33
   Met Gly Cys Cys Gly Cys Ser Gly Gly Cys Gly Ser Gly Cys Gly Gly
   1               5                   10                  15
   Cys Gly Ser Gly Cys Gly Gly Cys Gly Ser Ser Cys Cys Val Pro Ile
               20                  25                  30
   Cys Cys Cys Lys Pro Val Cys Cys Cys Val Pro Ala Cys Ser Cys Thr
           35                  40                  45
   Ser Cys Gly Ser Cys Gly Gly Ser Lys Gly Cys Cys Gly Ser Cys Gly
       50                  55                  60
   Gly Ser Lys Gly Gly Cys Gly Ser Cys Gly Gly Ser Lys Gly Gly Cys
   65                  70                  75                  80
   Gly Ser Cys Gly Cys Ser Gln Cys Ser Cys Cys Lys Pro Cys Tyr Cys
                   85                  90                  95
   Ser Ser Gly Cys Gly Ser Ser Cys Cys Gln Ser Ser Cys Cys Lys Pro
               100                 105                 110
   Cys Cys Ser Gln Ala Ser Cys Cys Val Pro Ile Cys Cys Gln Cys Lys
           115                 120                 125
   Ile
<210> 34
   <211> 165
   <212> PRT
   <213> Homo sapiens
<400> 34
   Met Gly Cys Cys Gly Cys Ser Glu Gly Cys Gly Ser Gly Cys Gly Gly
   1 5 10 15
   Cys Gly Ser Gly Cys Gly Gly Cys Gly Ser Gly Cys Gly Gly Cys Gly
   20 25 30
   Ser Ser Cys Cys Val Pro Val Cys Cys Cys Lys Pro Val Cys Cys Cys
   35 40 45
   Val Pro Ala Cys Ser Cys Ser Ser Cys Gly Ser Cys Gly Gly Ser Lys
   50 55 60
   Gly Gly Cys Gly Ser Cys Gly Gly Ser Lys Gly Gly Cys Gly Ser Cys
   65 70 75 80
   Gly Gly Ser Lys Gly Gly Cys Gly Ser Cys Gly Cys Ser Gln Cys Ser
   85 90 95
   Cys Tyr Lys Pro Cys Cys Cys Ser Ser Gly Cys Gly Ser Ser Cys Cys
   100 105 110
   Gln Ser Ser Cys Cys Lys Pro Cys Cys Cys Gln Ser Ser Cys Cys Lys
   115 120 125
   Pro Cys Cys Cys Ser Ser Gly Cys Gly Ser Ser Cys Cys Gln Ser Ser
   130 135 140
   Cys Cys Asn Pro Cys Cys Ser Gln Ser Ser Cys Cys Val Pro Val Cys
   145 150 155 160
   Cys Gln Cys Lys Ile
   165
<210> 35
   <211> 187
   <212> PRT
   <213> Homo sapiens
<400> 35
   Met Gly Cys Cys Gly Cys Ser Gly Gly Cys Gly Ser Gly Cys Gly Gly
   1 5 10 15
   Cys Gly Ser Gly Cys Gly Gly Cys Gly Ser Ser Cys Cys Val Pro Ile
   20 25 30
   Cys Cys Cys Lys Pro Val Cys Cys Cys Val Pro Ala Cys Ser Cys Ser
   35 40 45
   Ser Cys Gly Ser Cys Gly Gly Ser Lys Gly Gly Arg Gly Ser Cys Gly
   50 55 60
   Gly Ser Lys Gly Asp Cys Gly Ser Cys Gly Gly Ser Lys Gly Gly Cys
   65 70 75 80
   Gly Ser Cys Gly Cys Ser Gln Cys Ser Cys Tyr Lys Pro Cys Cys Cys
   85 90 95
   Ser Ser Gly Cys Gly Ser Ser Cys Cys Gln Ser Ser Cys Cys Lys Pro
   100 105 110
   Cys Cys Ser Gln Ser Ser Cys Cys Lys Pro Cys Ser Cys Ser Ser Gly
   115 120 125
   Cys Gly Ser Ser Cys Cys Gln Ser Ser Cys Cys Lys Pro Cys Cys Ser
   130 135 140
   Gln Ser Ser Cys Cys Lys Pro Cys Cys Cys Ser Ser Gly Cys Gly Ser
   145 150 155 160
   Ser Cys Cys Gln Ser Ser Cys Cys Lys Pro Cys Cys Ser Gln Ser Ser
   165 170 175
   Cys Cys Val Pro Ile Cys Cys Gln Cys Lys Ile
   180 185
<210> 36
   <211> 169
   <212> PRT
   <213> Homo sapiens
<400> 36
   Met Gly Cys Cys Gly Cys Ser Gly Gly Cys Gly Ser Ser Cys Gly Gly
   1 5 10 15
   Cys Asp Ser Ser Cys Gly Ser Cys Gly Ser Gly Cys Arg Gly Cys Gly
   20 25 30
   Pro Ser Cys Cys Ala Pro Val Tyr Cys Cys Lys Pro Val Cys Cys Cys
   35 40 45
   Val Pro Ala Cys Ser Cys Ser Ser Cys Gly Lys Arg Gly Cys Gly Ser
   50 55 60
   Cys Gly Gly Ser Lys Gly Gly Cys Gly Ser Cys Gly Cys Ser Gln Cys
   65 70 75 80
   Ser Cys Cys Lys Pro Cys Cys Cys Ser Ser Gly Cys Gly Ser Ser Cys
   85 90 95
   Cys Gln Cys Ser Cys Cys Lys Pro Tyr Cys Ser Gln Cys Ser Cys Cys
   100 105 110
   Lys Pro Cys Cys Ser Ser Ser Gly Arg Gly Ser Ser Cys Cys Gln Ser
   115 120 125
   Ser Cys Cys Lys Pro Cys Cys Ser Ser Ser Gly Cys Gly Ser Ser Cys
   130 135 140
   Cys Gln Ser Ser Cys Cys Lys Pro Cys Cys Ser Gln Ser Arg Cys Cys
   145 150 155 160
   Val Pro Val Cys Tyr Gln Cys Lys Ile
   165
<210> 37
   <211> 202
   <212> PRT
   <213> Homo sapiens
<400> 37
   Met Gly Cys Cys Gly Cys Ser Gly Gly Cys Gly Ser Gly Cys Gly Gly
   1               5                   10                  15
   Cys Gly Ser Gly Cys Gly Gly Cys Gly Ser Gly Cys Gly Gly Tyr Gly
               20                  25                  30
   Ser Gly Cys Gly Gly Cys Gly Ser Ser Cys Cys Val Pro Val Cys Cys
           35                  40                  45
   Cys Lys Pro Val Cys Cys Cys Val Pro Ala Cys Ser Cys Ser Ser Cys
       50                  55                  60
   Gly Ser Cys Gly Gly Ser Lys Gly Asp Cys Gly Ser Cys Gly Gly Ser
   65                  70                  75                  80
   Lys Gly Gly Cys Gly Ser Cys Gly Gly Ser Lys Gly Gly Cys Gly Ser
                   85                  90                  95
   Cys Gly Gly Ser Lys Gly Gly Cys Gly Ser Cys Gly Gly Ser Lys Gly
               100                 105                 110
   Gly Cys Gly Ser Cys Gly Gly Ser Lys Gly Gly Cys Gly Ser Cys Gly
           115                 120                 125
   Gly Ser Lys Gly Gly Cys Gly Ser Cys Gly Cys Ser Gln Cys Asn Cys
       130                 135                 140
   Cys Lys Pro Cys Cys Cys Ser Ser Gly Cys Gly Ser Cys Cys Gln Ser
   145                 150                 155                 160
   Ser Cys Cys Asn Pro Cys Cys Cys Gln Ser Ser Cys Cys Val Pro Val
                   165                 170                 175
   Cys Cys Gln Ser Ser Cys Cys Lys Pro Cys Cys Cys Gln Ser Ser Cys
               180                 185                 190
   Cys Val Pro Val Cys Cys Gln Cys Lys Ile
           195                 200
<210> 38
   <211> 156
   <212> PRT
   <213> Homo sapiens
<400> 38
   Met Gly Cys Cys Gly Cys Ser Gly Gly Cys Gly Ser Gly Cys Gly Gly
   1               5                   10                  15
   Cys Gly Ser Gly Ser Gly Gly Cys Gly Ser Gly Cys Gly Gly Cys Gly
               20                  25                  30
   Ser Ser Cys Cys Val Pro Ile Cys Cys Cys Lys Pro Val Cys Cys Cys
           35                  40                  45
   Val Pro Ala Cys Ser Cys Ser Ser Cys Gly Ser Cys Gly Gly Ser Lys
       50                  55                  60
   Gly Gly Cys Gly Ser Cys Gly Ser Ser Lys Gly Gly Cys Gly Ser Cys
   65                  70                  75                  80
   Gly Cys Ser Gln Ser Asn Cys Cys Lys Pro Cys Cys Ser Ser Ser Gly
                   85                  90                  95
   Cys Gly Ser Phe Cys Cys Gln Ser Ser Cys Ser Lys Pro Cys Cys Cys
               100                 105                 110
   Gln Ser Ser Cys Cys Gln Ser Ser Cys Cys Lys Pro Cys Cys Cys Gln
           115                 120                 125
   Ser Ser Cys Cys Gln Ser Ser Cys Phe Lys Pro Cys Cys Cys Gln Ser
       130                 135                 140
   Ser Cys Cys Val Pro Val Cys Cys Gln Cys Lys Ile
   145                 150                 155
<210> 39
   <211> 87
   <212> PRT
   <213> Homo sapiens
<400> 39
   Met Thr Arg Tyr Phe Cys Cys Gly Ser Tyr Phe Pro Gly Tyr Pro Ile
   1 5 10 15
   Tyr Gly Thr Asn Phe His Gly Thr Phe Arg Ala Thr Pro Leu Asn Cys
   20 25 30
   Val Val Pro Leu Gly Ser Pro Leu Asn Tyr Gly Cys Gly Cys Asn Gly
   35 40 45
   Tyr Ser Ser Leu Gly Tyr Ser Phe Gly Gly Ser Asn Ile Asn Asn Leu
   50 55 60
   Gly Gly Cys Tyr Gly Gly Ser Phe Tyr Arg Pro Trp Gly Ser Gly Ser
   65 70 75 80
   Gly Phe Gly Tyr Ser Thr Tyr
   85
<210> 40
   <211> 63
   <212> PRT
   <213> Homo sapiens
<400> 40
   Met Leu Cys Asp Asn Phe Pro Gly Ala Val Phe Pro Gly Cys Tyr Trp
   1 5 10 15
   Gly Ser Tyr Gly Tyr Pro Leu Gly Tyr Ser Val Gly Cys Gly Tyr Gly
   20 25 30
   Ser Thr Tyr Ser Pro Val Gly Tyr Gly Phe Gly Tyr Gly Tyr Asn Gly
   35 40 45
   Cys Gly Ala Phe Gly Tyr Arg Arg Tyr Ser Pro Phe Ala Leu Tyr
   50 55 60
<210> 41
   <211> 174
   <212> PRT
   <213> Homo sapiens
<400> 41
<210> 42
   <211> 159
   <212> PRT
   <213> Homo sapiens
<400> 42
   Met Thr His Cys Cys Ser Pro Cys Cys Gln Pro Thr Cys Cys Arg Thr
   1               5                   10                  15
   Thr Cys Trp Gln Pro Thr Thr Val Thr Thr Cys Ser Ser Thr Pro Cys
               20                  25                  30
   Cys Gln Pro Ser Cys Cys Val Ser Ser Cys Cys Gln Pro Cys Cys His
           35                  40                  45
   Pro Thr Cys Cys Gln Asn Thr Cys Cys Arg Thr Thr Cys Cys Gln Pro
       50                  55                  60
   Ile Cys Val Thr Ser Cys Cys Gln Pro Ser Cys Cys Ser Thr Pro Cys
   65 70 75 80
   Cys Gln Pro Thr Cys Cys Gly Ser Ser Cys Gly Gln Ser Ser Ser Cys
   85 90 95
   Ala Pro Val Tyr Cys Arg Arg Thr Cys Tyr His Pro Thr Ser Val Cys
   100 105 110
   Leu Pro Gly Cys Leu Asn Gln Ser Cys Gly Ser Asn Cys Cys Gln Pro
   115 120 125
   Cys Cys Arg Pro Ala Cys Cys Glu Thr Thr Cys Cys Arg Thr Thr Cys
   130 135 140
   Phe Gln Pro Thr Cys Val Tyr Ser Cys Cys Gln Pro Ser Cys Cys
   145 150 155
<210> 43
   <211> 154
   <212> PRT
   <213> Homo sapiens
<400> 43
   Met Thr His Cys Cys Ser Pro Cys Cys Gln Pro Thr Cys Cys Arg Thr
   1 5 10 15
   Thr Cys Cys Arg Thr Thr Cys Trp Lys Pro Thr Thr Val Thr Thr Cys
   20 25 30
   Ser Ser Thr Pro Cys Cys Gln Pro Ser Cys Cys Val Ser Ser Cys Cys
   35 40 45
   Gln Pro Cys Cys Arg Pro Thr Cys Cys Gln Asn Thr Cys Cys Gln Pro
   50 55 60
   Thr Cys Val Thr Ser Cys Cys Gln Pro Ser Cys Cys Ser Thr Pro Cys
   65 70 75 80
   Cys Gln Pro Thr Cys Cys Gly Ser Ser Cys Asp Gln Ser Ser Ser Cys
   85 90 95
   Ala Pro Val Tyr Cys Arg Arg Thr Cys Tyr Tyr Pro Thr Thr Val Cys
   100 105 110
   Leu Pro Gly Cys Leu Asn Gln Ser Cys Gly Ser Asn Cys Cys Gln Pro
   115 120 125
   Cys Cys Arg Pro Ala Cys Cys Glu Thr Thr Cys Phe Gln Pro Thr Cys
   130 135 140
   Val Tyr Ser Cys Cys Gln Pro Phe Cys Cys
   145 150
<210> 44
   <211> 111
   <212> PRT
   <213> Homo sapiens
<400> 44
   Thr Thr Cys Cys Gln Pro Thr Cys Leu Thr Ser Cys Cys Gln Pro Ser
   1 5 10 15
   Cys Cys Ser Thr Thr Cys Cys Gln Pro Ile Cys Cys Gly Ser Ser Cys
   20 25 30
   Cys Gly Gln Thr Ser Cys Gly Ser Ser Cys Gly Gln Ser Ser Ser Cys
   35 40 45
   Ala Pro Val Tyr Cys Arg Arg Thr Cys Tyr Tyr Pro Thr Thr Val Cys
   50 55 60
   Leu Pro Gly Cys Leu Asn Gln Ser Cys Gly Ser Ser Cys Cys Gln Pro
   65 70 75 80
   Cys Cys Arg Pro Ala Cys Cys Glu Thr Thr Cys Cys Arg Thr Thr Cys
   85 90 95
   Phe Gln Pro Thr Cys Val Ser Ser Cys Cys Gln Pro Ser Cys Cys
   100 105 110
<210> 45
   <211> 159
   <212> PRT
   <213> Homo sapiens
<400> 45
   Met Thr His Cys Cys Ser Pro Cys Cys Gln Pro Thr Cys Cys Arg Thr
   1               5                   10                  15
   Thr Cys Trp Lys Pro Thr Thr Val Thr Thr Cys Ser Ser Thr Pro Cys
               20                  25                  30
   Cys Gln Pro Ser Cys Cys Val Ser Ser Cys Cys Gln Pro Cys Cys Arg
           35                  40                  45
   Pro Thr Cys Cys Gln Asn Thr Cys Cys Gln Pro Ile Cys Val Thr Ser
       50                  55                  60
   Cys Cys Gln Pro Ser Cys Cys Ser Thr Pro Cys Cys Gln Pro Thr Cys
   65                  70                  75                  80
   Cys Gly Gln Thr Ser Cys Gly Ser Ser Cys Gly Gln Ser Ser Ser Cys
                   85                  90                  95
   Ala Pro Val Tyr Cys Arg Arg Thr Cys Tyr His Pro Thr Thr Val Cys
               100                 105                 110
   Leu Pro Gly Cys Leu Asn Gln Ser Cys Gly Ser Ser Cys Cys Gln Pro
           115                 120                 125
   Cys Cys Arg Pro Ala Cys Cys Glu Thr Thr Cys Cys Arg Thr Thr Cys
       130                 135                 140
   Phe Gln Pro Thr Cys Val Tyr Ser Cys Cys Gln Pro Ser Cys Cys
   145                 150                 155
<210> 46
   <211> 154
   <212> PRT
   <213> Homo sapiens
<400> 46
   Met Thr His Cys Cys Ser Pro Cys Cys Gln Pro Thr Cys Cys Arg Thr
   1               5                   10                  15
   Thr Cys Cys Arg Thr Thr Cys Trp Lys Pro Thr Thr Val Thr Thr Cys
               20                  25                  30
   Ser Ser Thr Pro Cys Cys Gln Pro Ser Cys Cys Val Ser Ser Cys Cys
           35                  40                  45
   Gln Pro Cys Cys Arg Pro Ala Cys Cys Gln Asn Thr Cys Cys Arg Thr
       50                  55                  60
   Thr Cys Cys Gln Pro Thr Cys Leu Ser Ser Cys Cys Gly Gln Thr Ser
   65                  70                  75                  80
   Cys Gly Ser Ser Cys Gly Gln Ser Ser Ser Cys Ala Pro Val Tyr Cys
                   85                  90                  95
   Arg Arg Thr Cys Tyr Tyr Pro Thr Thr Val Cys Leu Pro Gly Cys Leu
               100                 105                 110
   Asn Gln Ser Cys Gly Ser Ser Cys Cys Gln Pro Cys Cys Arg Pro Ala
           115                 120                 125
   Cys Cys Glu Thr Thr Cys Cys Arg Thr Thr Cys Phe Gln Pro Thr Cys
       130                 135                 140
   Val Ser Ser Cys Cys Gln Pro Ser Cys Cys
   145                 150
<210> 47
   <211> 282
   <212> PRT
   <213> Homo sapiens
<400> 47
   Met Ala Ala Ser Thr Met Ser Val Cys Ser Ser Ala Cys Ser Asp Ser
   1               5                   10                  15
   Trp Gln Val Asp Ala Cys Pro Glu Ser Cys Cys Glu Pro His Cys Cys
               20                  25                  30
   Ala Leu Ser Cys Cys Ala Pro Ala Pro Cys Leu Thr Leu Val Cys Thr
           35                  40                  45
   Pro Val Ser Arg Val Ser Ser Pro Cys Cys Gln Ala Ala Cys Glu Pro
       50                  55                  60
   Ser Pro Cys Gln Ser Gly Cys Thr Ser Ser Cys Thr Pro Ser Cys Cys
   65                  70                  75                  80
   Gln Gln Ser Ser Cys Gln Pro Ala Cys Cys Thr Ser Ser Pro Cys Gln
                   85                  90                  95
   Gln Ala Cys Cys Val Pro Val Cys Cys Lys Pro Val Cys Cys Leu Pro
               100                 105                 110
   Thr Cys Ser Lys Asp Ser Ser Ser Cys Cys Gln Gln Ser Ser Cys Gln
           115                 120                 125
   Pro Thr Cys Cys Ala Ser Ser Ser Ser Gln Gln Ser Cys Cys Val Pro
       130                 135                 140
   Val Cys Cys Lys Pro Val Cys Tyr Val Pro Thr Cys Ser Glu Asp Ser
   145                 150                 155                 160
   Ser Ser Cys Cys Gln Gln Ser Ser Cys His Pro Ala Cys Cys Thr Ser
                   165                 170                 175
   Ser Pro Cys Gln Gln Ala Cys Cys Val Pro Val Arg Cys Lys Pro Val
               180                 185                 190
   Cys Cys Lys Pro Ile Cys Cys Val Pro Val Cys Ser Gly Ala Ser Thr
           195                 200                 205
   Ser Cys Cys Gln Gln Ser Ser Cys Gln Pro Ala Cys Cys Thr Thr Ser
       210                 215                 220
   Cys Cys Arg Pro Ser Ser Ser Val Ser Leu Leu Cys Arg Pro Val Cys
   225                 230                 235                 240
   Arg Pro Ala Cys Cys Met Pro Val Ser Ser Cys Cys Ala Pro Ala Ser
                   245                 250                 255
   Ser Cys Gln Ala Ser Cys Cys Arg Pro Ala Ser Cys Val Ser Leu Leu
               260                 265                 270
   Cys Arg Pro Ala Cys Ser Arg Pro Ala Cys
           275                 280
<210> 48
   <211> 255
   <212> PRT
   <213> Homo sapiens
<400> 48
   Met Ala Ala Ser Thr Met Ser Ile Cys Ser Ser Ala Cys Thr Asn Ser
   1               5                   10                  15
   Trp Gln Val Asp Asp Cys Pro Glu Ser Cys Cys Glu Leu Pro Cys Gly
               20                  25                  30
   Thr Pro Ser Cys Cys Ala Pro Ala Pro Cys Leu Thr Leu Val Cys Thr
           35                  40                  45
   Pro Val Ser Cys Val Ser Ser Pro Cys Cys Gln Ala Ala Cys Glu Pro
       50                  55                  60
   Ser Ala Cys Gln Ser Gly Cys Thr Ser Ser Cys Thr Pro Ser Cys Cys
   65                  70                  75                  80
   Gln Gln Ser Ser Cys Gln Pro Ala Cys Cys Thr Ser Ser Pro Cys Gln
                   85                  90                  95
   Gln Ala Cys Cys Val Pro Val Cys Cys Lys Pro Val Cys Cys Val Pro
               100                 105                 110
   Val Cys Cys Gly Ala Ser Ser Cys Cys Gln Gln Ser Ser Cys Gln Pro
           115                 120                 125
   Ala Cys Cys Ala Ser Ser Ser Cys Gln Gln Ser Cys Arg Val Pro Val
       130                 135                 140
   Cys Cys Lys Ala Val Cys Cys Val Pro Thr Cys Ser Glu Ser Ser Ser
   145                 150                 155                 160
   Ser Cys Cys Gln Gln Ser Ser Cys Gln Pro Ala Cys Cys Thr Ser Ser
                   165                 170                 175
   Pro Cys Gln Gln Ser Cys Cys Val Ser Val Cys Cys Lys Pro Val Cys
               180                 185                 190
   Cys Lys Ser Ile Cys Cys Val Pro Val Cys Ser Gly Ala Ser Ser Pro
           195                 200                 205
   Cys Cys Gln Gln Ser Ser Cys Gln Pro Ala Cys Cys Thr Ser Ser Cys
       210                 215                 220
   Cys Arg Pro Ser Ser Ser Val Ser Leu Leu Cys Arg Pro Val Cys Ser
   225                 230                 235                 240
   Arg Pro Ala Ser Cys Ser Phe Ser Ser Gly Gln Lys Ser Ser Cys
                   245                 250                 255
<210> 49
   <211> 221
   <212> PRT
   <213> Homo sapiens
<400> 49
   Met Ala Thr Ser Thr Met Ser Val Cys Ser Ser Ala Tyr Ser Asp Ser
   1               5                   10                  15
   Trp Gln Val Asp Ala Cys Pro Glu Ser Cys Cys Glu Pro Pro Cys Cys
               20                  25                  30
   Ala Thr Ser Cys Cys Ala Pro Ala Pro Cys Leu Thr Leu Val Cys Thr
           35                  40                  45
   Pro Val Ser Cys Val Ser Ser Pro Cys Cys Gln Ala Ala Cys Glu Pro
       50                  55                  60
   Ser Pro Cys Gln Ser Gly Cys Thr Ser Ser Cys Thr Pro Ser Cys Cys
   65                  70                  75                  80
   Gln Gln Ser Ser Cys Gln Pro Ala Cys Cys Thr Ser Ser Pro Cys Gln
                   85                  90                  95
   Gln Ala Cys Cys Val Pro Val Cys Cys Lys Pro Val Cys Cys Val Pro
               100                 105                 110
   Val Cys Cys Lys Pro Val Cys Cys Lys Pro Ile Cys Cys Val Pro Val
           115                 120                 125
   Cys Ser Gly Ala Ser Ser Ser Cys Cys Gln Gln Ser Ser Arg Gln Pro
       130                 135                 140
   Ala Cys Cys Thr Thr Ser Cys Cys Arg Pro Ser Ser Ser Val Ser Leu
   145                 150                 155                 160
   Leu Cys Arg Pro Val Cys Arg Ser Thr Cys Cys Val Pro Ile Pro Ser
                   165                 170                 175
   Cys Cys Ala Pro Ala Ser Thr Cys Gln Pro Ser Cys Cys Arg Pro Ala
               180                 185                 190
   Ser Cys Val Ser Leu Leu Cys Arg Pro Thr Cys Ser Arg Leu Ser Ser
                    195                 200                 205
   Ala Cys Cys Gly Leu Ser Ser Gly Gln Lys Ser Ser Cys
       210                 215                 220
<210> 50
   <211> 401
   <212> PRT
   <213> Homo sapiens
<400> 50
   Met Ser Val Cys Ser Ser Asp Leu Ser Tyr Ser Ser Arg Val Cys Leu
   1 5 10 15
   Pro Gly Ser Cys Asp Ser Cys Ser Asp Ser Trp Gln Val Asp Asp Cys
   20 25 30
   Pro Glu Ser Cys Cys Glu Pro Pro Cys Cys Ala Pro Ser Cys Cys Ala
   35 40 45
   Pro Ala Pro Cys Leu Ser Leu Val Cys Thr Pro Val Ser Arg Val Ser
   50 55 60
   Ser Pro Cys Cys Pro Val Thr Cys Glu Pro Ser Pro Cys Gln Ser Gly
   65 70 75 80
   Cys Thr Ser Ser Cys Thr Pro Ser Cys Cys Gln Gln Ser Ser Cys Gln
   85 90 95
   Leu Ala Cys Cys Ala Ser Ser Pro Cys Gln Gln Ala Cys Cys Val Pro
   100 105 110
   Val Cys Cys Lys Thr Val Cys Cys Lys Pro Val Cys Cys Val Pro Val
   115 120 125
   Cys Cys Gly Asp Ser Ser Cys Cys Gln Gln Ser Ser Cys Gln Ser Ala
   130 135 140
   Cys Cys Thr Ser Ser Pro Cys Gln Gln Ala Cys Cys Val Pro Ile Cys
   145 150 155 160
   Cys Lys Pro Val Cys Ser Gly Ile Ser Ser Ser Cys Cys Gln Gln Ser
   165 170 175
   Ser Cys Val Ser Cys Val Ser Ser Pro Cys Cys Gln Ala Val Cys Glu
   180 185 190
   Pro Ser Pro Cys Gln Ser Gly Cys Ile Ser Ser Cys Thr Pro Ser Cys
   195 200 205
   Cys Gln Gln Ser Ser Cys Gln Pro Ala Cys Cys Thr Ser Ser Ser Cys
   210 215 220
   Gln Gln Ala Cys Cys Val Pro Val Cys Cys Lys Thr Val Cys Cys Lys
   225 230 235 240
   Pro Val Cys Ser Glu Asp Ser Ser Ser Cys Cys Gln Gln Ser Ser Cys
   245 250 255
   Gln Pro Ala Cys Cys Thr Ser Ser Pro Cys Gln Gln Ala Cys Cys Val
   260 265 270
   Pro Val Cys Cys Lys Pro Val Cys Cys Lys Pro Val Gly Ser Val Pro
   275 280 285
   Ile Cys Ser Gly Ala Ser Ser Leu Cys Cys Gln Gln Ser Ser Cys Gln
   290 295 300
   Pro Ala Cys Cys Thr Ser Ser Gln Ser Gln Gln Gly Cys Cys Val Pro
   305 310 315 320
   Val Cys Cys Lys Pro Val Ser Cys Val Pro Val Cys Ser Gly Ala Ser
   325 330 335
   Ser Ser Cys Cys Gln Gln Ser Ser Cys Gln Pro Ala Cys Cys Thr Thr
   340 345 350
   Ser Cys Cys Arg Pro Ser Ser Ser Val Ser Leu Leu Cys Arg Pro Val
   355 360 365
   Cys Arg Pro Ala Cys Cys Val Pro Val Pro Ser Cys Cys Ala Pro Thr
   370 375 380
   Ser Ser Cys Gln Pro Ser Cys Cys Arg Pro Ala Ser Cys Val Ser Leu
   385 390 395 400
   Leu
<210> 51
   <211> 271
   <212> PRT
   <213> Homo sapiens
<400> 51
   Met Ala Ala Cys Thr Met Ser Val Cys Ser Ser Ala Cys Ser Asp Ser
   1 5 10 15
   Trp Arg Val Asp Asp Cys Pro Glu Ser Cys Cys Glu Pro Pro Cys Gly
   20 25 30
   Thr Ala Pro Cys Leu Thr Leu Val Cys Thr Pro Val Ser Cys Val Ser
   35 40 45
   Ser Pro Cys Cys Gln Ala Ala Cys Glu Pro Ser Pro Cys Gln Ser Gly
   50 55 60
   Cys Thr Ser Ser Cys Thr Pro Ser Cys Cys Gln Pro Ala Cys Cys Ala
   65 70 75 80
   Ser Ser Pro Cys Gln Gln Ala Cys Cys Val Pro Val Cys Cys Lys Pro
   85 90 95
   Val Cys Cys Leu Pro Thr Cys Ser Lys Asp Ser Ser Ser Cys Cys Gln
   100 105 110
   Gln Ser Ser Cys Gln Pro Thr Cys Cys Ala Ser Ser Ser Cys Gln Gln
   115 120 125
   Ser Cys Cys Val Pro Val Cys Cys Lys Pro Val Cys Cys Val Pro Thr
   130 135 140
   Cys Ser Glu Asp Ser Ser Ser Cys Cys Gln His Ser Ser Cys Gln Pro
   145 150 155 160
   Thr Cys Cys Thr Ser Ser Pro Cys Gln Gln Ser Cys Tyr Val Pro Val
   165 170 175
   Cys Cys Lys Pro Val Cys Phe Lys Pro Ile Cys Cys Val Pro Val Cys
   180 185 190
   Ser Gly Ala Ser Thr Ser Cys Cys Gln Gln Ser Ser Cys Gln Pro Ala
   195 200 205
   Cys Cys Thr Thr Ser Cys Cys Arg Pro Ser Ser Ser Val Ser Leu Leu
   210 215 220
   Cys Arg Pro Ile Cys Arg Pro Ala Cys Cys Val Pro Ile Ser Ser Cys
   225 230 235 240
   Cys Ala Pro Ala Ser Ser Tyr Gln Ala Ser Cys Cys Arg Pro Ala Ser
   245 250 255
   Cys Val Ser Leu Leu Cys Arg Pro Ala Cys Ser Arg Leu Ala Cys
   260 265 270
<210> 52
   <211> 365
   <212> PRT
   <213> Homo sapiens
<400> 52
   Met Ala Ala Ser Thr Met Ser Val Cys Ser Ser Asp Leu Ser Tyr Gly
   1 5 10 15
   Ser Arg Val Cys Leu Pro Gly Ser Cys Asp Ser Cys Ser Asp Ser Trp
   20 25 30
   Gln Val Asp Asp Cys Pro Glu Ser Cys Cys Glu Pro Pro Cys Cys Ala
   35 40 45
   Pro Ala Pro Cys Leu Ser Leu Val Cys Thr Pro Val Ser Arg Val Ser
   50 55 60
   Ser Pro Cys Cys Pro Val Thr Cys Glu Pro Ser Pro Cys Gln Ser Gly
   65 70 75 80
   Cys Thr Ser Ser Cys Thr Pro Ser Cys Cys Gln Gln Ser Ser Cys Gln
   85 90 95
   Leu Ala Cys Cys Ala Ser Ser Pro Cys Gln Gln Ala Cys Cys Val Pro
   100 105 110
   Val Cys Cys Lys Thr Val Cys Cys Lys Pro Val Cys Cys Val Ser Val
   115 120 125
   Cys Cys Gly Asp Ser Ser Cys Cys Gln Gln Ser Ser Cys Gln Ser Ala
   130 135 140
   Cys Cys Thr Ser Ser Pro Cys Gln Gln Ala Cys Cys Val Pro Val Cys
   145 150 155 160
   Cys Lys Pro Val Cys Ser Gly Ile Ser Ser Ser Cys Cys Gln Gln Ser
   165 170 175
   Ser Cys Val Ser Cys Val Ser Ser Pro Cys Cys Gln Ala Val Cys Glu
   180 185 190
   Pro Ser Pro Cys Gln Ser Gly Cys Thr Ser Ser Cys Thr Pro Ser Cys
   195 200 205
   Cys Gln Gln Ser Ser Cys Gln Pro Thr Cys Cys Thr Ser Ser Pro Cys
   210 215 220
   Gln Gln Ala Cys Cys Val Pro Val Cys Cys Val Pro Val Cys Cys Val
   225 230 235 240
   Pro Thr Cys Ser Glu Asp Ser Ser Ser Cys Cys Gln Gln Ser Ser Cys
   245 250 255
   Gln Pro Ala Cys Cys Thr Ser Ser Pro Cys Gln His Ala Cys Cys Val
   260 265 270
   Pro Val Cys Ser Gly Ala Ser Thr Ser Cys Cys Gln Gln Ser Ser Cys
   275 280 285
   Gln Pro Ala Cys Cys Thr Ala Ser Cys Cys Arg Pro Ser Ser Ser Val
       290                 295                 300
   Ser Leu Leu Cys His Pro Val Cys Lys Ser Thr Cys Cys Val Pro Val
   305                 310                 315                 320
   Pro Ser Cys Gly Ala Ser Ala Ser Ser Cys Gln Pro Ser Cys Cys Arg
                   325                 330                 335
   Thr Ala Ser Cys Val Ser Leu Leu Cys Arg Pro Met Cys Ser Arg Pro
               340                 345                 350
   Ala Cys Tyr Ser Leu Cys Ser Gly Gln Lys Ser Ser Cys
           355                 360                 365
<210> 53
   <211> 375
   <212> PRT
   <213> Homo sapiens
<400> 53
   Met Ala Ala Ser Thr Met Ser Val Cys Ser Ser Asp Leu Ser Tyr Gly
   1               5                   10                  15
   Ser Arg Val Cys Leu Pro Gly Ser Cys Asp Ser Cys Ser Asp Ser Trp
               20                  25                  30
   Gln Val Asp Asp Cys Pro Glu Ser Cys Cys Glu Pro Pro Cys Cys Ala
           35                  40                  45
   Pro Ser Cys Cys Ala Pro Ala Pro Cys Leu Ser Leu Val Cys Thr Pro
       50                  55                  60
   Val Ser Arg Val Ser Ser Pro Cys Cys Pro Val Thr Cys Glu Pro Ser
   65                  70                  75                  80
   Pro Cys Gln Ser Gly Cys Thr Ser Ser Cys Thr Pro Ser Cys Cys Gln
                   85                  90                  95
   Gln Ser Ser Cys Gln Leu Ala Cys Cys Ala Ser Ser Pro Cys Gln Gln
               100                 105                 110
   Ala Cys Cys Met Pro Val Cys Cys Lys Thr Val Cys Cys Lys Pro Val
           115                 120                 125
   Tyr Cys Val Pro Val Cys Ser Gly Asp Ser Ser Cys Cys Gln Gln Ser
       130                 135                 140
   Ser Cys Gln Ser Ala Cys Cys Thr Ser Ser Pro Cys Gln Gln Ala Cys
   145                 150                 155                 160
   Cys Val Pro Ile Cys Cys Lys Pro Val Cys Ser Gly Ile Ser Ser Ser
   165 170 175
   Cys Cys Gln Gln Ser Ser Cys Val Ser Cys Val Ser Ser Pro Cys Cys
   180 185 190
   Gln Ala Val Cys Glu Pro Ser Pro Cys Gln Ser Gly Cys Ile Ser Ser
   195 200 205
   Cys Thr Pro Ser Cys Cys Gln Gln Ser Ser Cys Gln Pro Ala Cys Cys
   210 215 220
   Thr Ser Ser Pro Cys Gln Gln Ala Cys Cys Val Pro Val Cys Cys Lys
   225 230 235 240
   Pro Val Cys Cys Val Pro Thr Cys Ser Asp Asp Ser Gly Ser Cys Cys
   245 250 255
   Gln Pro Ala Cys Cys Thr Ser Ser Gln Ser Gln Gln Gly Cys Cys Val
   260 265 270
   Pro Val Cys Cys Lys Pro Val Cys Cys Val Pro Val Cys Ser Gly Ala
   275 280 285
   Ser Ser Ser Cys Cys Gln Gln Ser Ser Cys Gln Pro Ala Cys Cys Thr
   290 295 300
   Thr Ser Cys Cys Arg Pro Ser Ser Ser Val Ser Leu Leu Cys Arg Pro
   305 310 315 320
   Val Cys Arg Pro Thr Cys Cys Val Pro Val Pro Ser Cys Cys Ala Pro
   325 330 335
   Thr Ser Ser Cys Gln Ala Ser Cys Cys Arg Pro Ala Ser Cys Val Ser
   340 345 350
   Leu Leu Cys Arg Pro Ala Cys Ser Arg Pro Ala Cys Cys Gly Pro Thr
   355 360 365
   Ser Thr Gln Lys Ser Ser Cys
   370 375
<210> 54
   <211> 259
   <212> PRT
   <213> Homo sapiens
<400> 54
   Met Ala Asp Ala Cys Cys Thr Arg Thr Tyr Val Ile Ala Ala Ser Thr
   1               5                   10                  15
   Met Ser Val Cys Ser Ser Asp Val Gly His Val Ser Arg Val Ser Ser
               20                  25                  30
   Pro Ser Thr Cys Thr Gly Ser Ser Trp Gln Val Asp Asn Cys Gln Glu
           35                  40                  45
   Ser Cys Cys Glu Pro Arg Ser Cys Ala Ser Ser Cys Cys Thr Pro Ser
       50                  55                  60
   Cys Cys Ala Pro Ala Pro Cys Leu Ala Leu Val Cys Ala Pro Val Ser
   65                  70                  75                  80
   Cys Glu Pro Ser Pro Cys Gln Ser Gly Cys Thr Asp Ser Cys Thr Pro
                   85                  90                  95
   Ser Cys Cys Gln Gln Ser Ser Cys Gln Pro Ala Cys Cys Thr Ser Ser
               100                 105                 110
   Pro Cys Gln Gln Ala Cys Cys Val Pro Val Cys Cys Lys Ser Asn Cys
           115                 120                 125
   Cys Lys Pro Val Cys Cys Val Ser Ile Cys Ser Gly Ala Ser Ser Pro
       130                 135                 140
   Cys Cys Gln Gln Ser Ser Cys Gln Ser Ala Cys Cys Thr Phe Ser Pro
   145                 150                 155                 160
   Cys Gln Gln Ala Cys Cys Val Pro Ile Cys Cys Lys Pro Ile Cys Cys
                   165                 170                 175
   Val Pro Val Cys Ser Gly Ala Ser Ser Leu Cys Cys Gln Lys Ser Ser
               180                 185                 190
   Cys Gln Pro Ala Cys Cys Thr Thr Ser Cys Cys Arg Pro Ser Ser Ser
           195                 200                 205
   Val Ser Leu Leu Cys Arg Pro Val Cys Arg Pro Ala Cys Cys Val Pro
       210                 215                 220
   Val Pro Ser Cys Cys Val Pro Ala Ser Ser Cys Gln Pro Ser Cys Cys
   225                 230                 235                 240
   His Pro Ala Ser Cys Leu Ser Phe Leu Cys Arg Pro Ala Cys Ser Arg
                   245                 250                 255
   Leu Ala Cys
<210> 55
   <211> 292
   <212> PRT
   <213> Homo sapiens
<400> 55
   Met Ala Ala Ser Thr Met Ser Ile Arg Ser Ser Ala Tyr Ser Asp Ser
   1               5                   10                  15
   Trp Gln Val Asp Asp Cys Pro Glu Ser Cys Cys Glu Pro Pro Cys Cys
               20                  25                  30
   Ala Thr Ser Cys Cys Ala Pro Ala Pro Cys Leu Thr Leu Val Cys Thr
           35                  40                  45
   Pro Val Ser Arg Val Ser Ser Pro Cys Cys Gln Val Thr Cys Glu Pro
       50                  55                  60
   Ser Pro Cys Gln Ser Gly Cys Thr Ser Ser Cys Thr Pro Ser Cys Cys
   65                  70                  75                  80
   Gln Gln Ser Ser Cys Gln Pro Ala Tyr Cys Thr Ser Ser Pro Cys Gln
                   85                  90                  95
   Gln Ala Cys Cys Val Pro Val Cys Cys Lys Pro Val Cys Cys Val Pro
               100                 105                 110
   Val Cys Cys Gly Ala Ser Ser Cys Cys Gln Gln Ser Ser Tyr Gln Pro
           115                 120                 125
   Ala Cys Cys Ala Ser Ser Ser Cys Gln Pro Ala Cys Cys Val Pro Val
       130                 135                 140
   Cys Cys Lys Pro Val Cys Cys Ala Pro Thr Cys Ser Glu Asp Ser Tyr
   145                 150                 155                 160
   Ser Cys Cys Gln Gln Ser Ser Cys Gln Pro Ala Cys Cys Thr Ser Ser
                   165                 170                 175
   Pro Cys Gln Gln Ser Tyr Cys Val Pro Val Cys Cys Lys Pro Val Cys
               180                 185                 190
   Cys Lys Pro Ile Cys Cys Val Pro Val Cys Ser Gly Ala Ser Ser Leu
           195                 200                 205
   Cys Cys Gln Gln Ser Gly Cys Gln Pro Ala Cys Cys Thr Thr Ser Cys
       210                 215                 220
   Cys Arg Pro Ser Ser Ser Val Ser Leu Leu Cys Arg Pro Val Cys Arg
   225                 230                 235                 240
   Pro Ala Cys Cys Val Pro Val Ser Ser Cys Cys Ala Pro Thr Ser Ser
                   245                 250                 255
   Arg Gln Pro Ser Tyr Cys Arg Gln Ala Ser Cys Val Ser Leu Leu Cys
               260                 265                 270
   Arg Pro Val Cys Ser Arg Pro Ala Cys Tyr Ser Phe Ser Ser Gly Gln
   275 280 285
   Lys Ser Ser Cys
   290
<210> 56
   <211> 251
   <212> PRT
   <213> Homo sapiens
<400> 56
   Met Ala Ala Ser Thr Met Ser Ile Cys Ser Ser Ala Cys Thr Asp Ser
   1 5 10 15
   Trp Arg Val Val Asp Cys Pro Glu Ser Cys Cys Glu Pro Cys Cys Cys
   20 25 30
   Ala Pro Ala Pro Ser Leu Thr Leu Val Cys Thr Pro Val Ser Cys Val
   35 40 45
   Ser Ser Pro Cys Cys Gln Thr Ala Cys Glu Pro Ser Ala Cys Gln Ser
   50 55 60
   Gly Tyr Thr Ser Ser Cys Thr Thr Pro Cys Tyr Gln Gln Ser Ser Cys
   65 70 75 80
   Gln Pro Asp Cys Cys Thr Ser Ser Pro Cys Gln Gln Ala Cys Cys Val
   85 90 95
   Pro Val Cys Cys Val Pro Val Cys Cys Val Pro Val Cys Asn Lys Pro
   100 105 110
   Val Cys Phe Val Pro Thr Cys Ser Glu Ser Ser Pro Ser Cys Cys Gln
   115 120 125
   Gln Ser Ser Cys Gln Pro Thr Cys Cys Thr Ser Ser Pro Cys Gln Gln
   130 135 140
   Ala Cys Cys Val Pro Val Cys Ser Lys Ser Val Cys Tyr Val Pro Val
   145 150 155 160
   Cys Ser Gly Ala Ser Thr Ser Cys Cys Gln Gln Ser Ser Cys Gln Pro
   165 170 175
   Ala Cys Cys Thr Ala Ser Cys Cys Arg Pro Ser Ser Ser Val Ser Leu
   180 185 190
   Leu Cys His Pro Val Cys Lys Ser Thr Cys Cys Val Pro Val Pro Ser
   195 200 205
   Cys Gly Ala Ser Ala Ser Ser Cys Gln Pro Ser Cys Cys Arg Thr Ala
   210 215 220
   Ser Cys Val Ser Leu Leu Cys Arg Pro Val Cys Ser Arg Pro Ala Cys
   225 230 235 240
   Tyr Ser Leu Cys Ser Gly Gln Lys Ser Ser Cys
   245 250
<210> 57
   <211> 298
   <212> PRT
   <213> Homo sapiens
<400> 57
   Met Ala Ala Ser Thr Met Ser Val Cys Ser Ser Ala Tyr Ser Asp Ser
   1               5                   10                  15
   Trp Gln Val Asp Asp Cys Pro Glu Ser Cys Cys Glu Pro Pro Cys Ser
               20                  25                  30
   Ala Pro Ser Cys Cys Ala Pro Ala Pro Ser Leu Ser Leu Val Cys Thr
           35                  40                  45
   Pro Val Ser Cys Val Ser Ser Pro Cys Cys Gln Ala Ala Cys Glu Pro
       50                  55                  60
   Ser Ala Cys Gln Ser Gly Cys Thr Ser Ser Cys Thr Pro Ser Cys Cys
   65                  70                  75                  80
   Gln Gln Ser Ser Cys Gln Pro Ala Cys Cys Thr Ser Ser Pro Cys Gln
                   85                  90                  95
   Gln Ala Cys Cys Val Pro Val Cys Cys Lys Thr Val Cys Cys Lys Pro
               100                 105                 110
   Val Cys Cys Val Pro Val Cys Cys Gly Ala Ala Ser Ser Cys Cys Arg
           115                 120                 125
   Gln Ser Ser Cys Gln Pro Ala Cys Cys Ala Ser Ser Ser Cys Gln Pro
       130                 135                 140
   Ala Cys Cys Val Pro Val Cys Cys Lys Pro Val Cys Cys Val Ser Thr
   145                 150                 155                 160
   Cys Ser Glu Asp Ser Ser Ser Cys Cys Gln Gln Ser Ser Cys Gln Pro
                   165                 170                 175
   Ala Cys Cys Thr Ser Ser Ser Tyr Gln Gln Ala Cys Cys Val Pro Val
               180                 185                 190
   Cys Cys Lys Thr Val Tyr Cys Lys Pro Ile Cys Cys Val Pro Val Cys
   195 200 205
   Ser Arg Ala Ser Ser Ser Arg Cys Gln Gln Pro Ser Cys Gln Pro Ala
   210 215 220
   Cys Cys Thr Thr Ser Cys Cys Arg Pro Ser Ser Ser Val Ser Leu Leu
   225 230 235 240
   Cys His Pro Val Cys Arg Ser Thr Cys Cys Val Pro Val Ser Ser Cys
   245 250 255
   Cys Ala Pro Thr Ser Ser Cys Gln Ser Ser Cys Cys Cys Pro Ala Ser
   260 265 270
   Cys Val Ser Leu Leu Cys Arg Pro Ala Ser Ser Arg Leu Ala Cys Tyr
   275 280 285
   Ser Leu Cys Ser Gly Lys Lys Ser Ser Cys
   290 295
<210> 58
   <211> 245
   <212> PRT
   <213> Homo sapiens
<400> 58
   Met Ser Val Cys Ser Ser Asp Leu Ser Tyr Gly Ser Arg Val Cys Leu
   1               5                   10                  15
   Pro Gly Ser Cys Asp Ser Cys Ser Asp Ser Trp Gln Val Asp Asp Cys
               20                  25                  30
   Pro Glu Ser Cys Cys Glu Pro Pro Cys Cys Ala Pro Ala Pro Cys Leu
           35                  40                  45
   Ser Leu Val Cys Thr Pro Val Ser Arg Val Ser Ser Pro Cys Cys Arg
       50                  55                  60
   Val Thr Cys Glu Pro Ser Pro Cys Gln Ser Gly Cys Thr Ser Ser Cys
   65                  70                  75                  80
   Thr Pro Ser Cys Cys Gln Gln Ser Ser Cys Gln Pro Ala Cys Cys Thr
                   85                  90                  95
   Ser Ser Pro Cys Gln Gln Ala Cys Cys Val Pro Val Cys Cys Lys Thr
               100                 105                 110
   Val Cys Cys Lys Pro Val Cys Cys Met Pro Val Cys Cys Gly Pro Ser
           115                 120                 125
   Ser Ser Cys Cys Gln Gln Ser Ser Cys Gln Pro Ala Cys Cys Ile Ser
       130                 135                 140
   Ser Pro Cys Gln Gln Ser Cys Cys Val Pro Val Cys Cys Lys Pro Ile
   145                 150                 155                 160
   Cys Cys Val Pro Val Cys Ser Gly Ala Ser Ser Leu Cys Cys Gln Gln
                   165                 170                 175
   Ser Ser Cys Gln Pro Ala Cys Cys Thr Thr Ser Cys Cys Arg Pro Ser
               180                 185                 190
   Ser Ser Val Ser Leu Leu Cys Arg Pro Val Cys Arg Pro Ala Arg Arg
           195                 200                 205
   Val Pro Val Pro Ser Cys Cys Val Pro Thr Ser Ser Cys Gln Pro Ser
       210                 215                 220
   Cys Gly Arg Leu Ala Ser Cys Gly Ser Leu Leu Cys Arg Pro Thr Cys
   225                 230                 235                 240
   Ser Arg Leu Ala Cys
                   245
<210> 59
   <211> 163
   <212> PRT
   <213> Homo sapiens
<400> 59
   Met Ser Phe Asn Cys Ser Thr Arg Asn Cys Ser Ser Arg Pro Ile Gly
   1               5                   10                  15
   Gly Arg Cys Ile Val Pro Val Ala Gln Val Thr Thr Thr Ser Thr Thr
               20                  25                  30
   Asp Ala Asp Cys Leu Gly Gly Ile Cys Leu Pro Ser Ser Phe Gln Thr
           35                  40                  45
   Gly Ser Trp Leu Leu Asp His Cys Gln Glu Thr Cys Cys Glu Pro Thr
       50                  55                  60
   Ala Cys Gln Pro Thr Cys Tyr Arg Arg Thr Ser Cys Val Ser Asn Pro
   65                  70                  75                  80
   Cys Gln Val Thr Cys Ser Arg Gln Thr Thr Cys Ile Ser Asn Pro Cys
                   85                  90                  95
   Ser Thr Thr Tyr Ser Arg Pro Leu Thr Phe Val Ser Ser Gly Cys Gln
               100                 105                 110
   Pro Leu Gly Gly Ile Ser Ser Val Cys Gln Pro Val Gly Gly Ile Ser
           115                 120                 125
   Thr Val Cys Gln Pro Val Gly Gly Val Ser Thr Val Cys Gln Pro Ala
       130                 135                 140
   Cys Gly Val Ser Arg Thr Tyr Gln Gln Ser Cys Val Ser Ser Cys Arg
   145                 150                 155                 160
   Arg Thr Cys
<210> 60
   <211> 96
   <212> PRT
   <213> Homo sapiens
<400> 60
   Met Cys His Thr Ser Cys Ser Ser Gly Cys Gln Pro Ala Cys Cys Ala
   1               5                   10                  15
   Pro Ser Pro Cys Gln Ala Ser Cys Tyr Ile Pro Val Gly Cys Gln Ser
               20                  25                  30
   Ser Val Cys Val Pro Val Ser Phe Lys Pro Ala Val Cys Val Pro Val
           35                  40                  45
   Arg Cys Gln Ser Ser Val Cys Val Pro Val Ser Cys Arg Pro Val Val
       50                  55                  60
   Tyr Ala Ala Pro Ser Cys Gln Ser Ser Gly Cys Cys Gln Pro Ser Cys
   65                  70                  75                  80
   Thr Ser Val Leu Cys Arg Pro Ile Ser Cys Ser Thr Pro Ser Cys Cys
                   85                  90                  95
<210> 61
   <211> 146
   <212> PRT
   <213> Homo sapiens
<400> 61
   Met Cys His Thr Ser Cys Ser Ser Gly Cys Gln Pro Ala Cys Cys Ala
   1               5                   10                  15
   Pro Ser Pro Cys Gln Pro Ala Cys Cys Val Pro Ser Ser Cys Gln Ala
               20                  25                  30
   Ser Cys Cys Val Pro Val Gly Cys Gln Ser Ser Val Cys Val Pro Val
           35                  40                  45
   Ser Phe Lys Pro Ala Val Cys Leu Pro Val Ser Cys Gln Ser Ser Val
       50                  55                  60
   Cys Val Pro Met Ser Phe Lys Ser Ala Val Cys Val Pro Val Ser Cys
   65 70 75 80
   Gln Ser Ser Val Cys Val Pro Val Ser Cys Arg Pro Ile Val Cys Ala
   85 90 95
   Ala Pro Ser Cys Gln Ser Ser Leu Cys Val Pro Val Ser Cys Arg Pro
   100 105 110
   Val Val Tyr Ala Ala Pro Ser Cys Gln Ser Ser Gly Cys Cys Gln Pro
   115 120 125
   Ser Cys Thr Ser Val Leu Cys Arg Pro Ile Ser Tyr Ser Ile Ser Ser
   130 135 140
   Cys Cys
   145
<210> 62
   <211> 96
   <212> PRT
   <213> Homo sapiens
<400> 62
   Met Cys His Thr Ser Cys Ser Pro Ala Cys Gln Pro Thr Cys Cys Ile
   1 5 10 15
   Arg Ser Pro Cys Gln Ala Ser Cys Tyr Val Pro Val Ser Cys Gln Ser
   20 25 30
   Ser Val Cys Met Pro Val Ser Cys Thr Arg Ile Val Cys Val Ala Pro
   35 40 45
   Ser Cys Gln Pro Ser Val Cys Val Pro Val Ser Cys Arg Pro Ile Ile
   50 55 60
   Tyr Val Thr Pro Ser Cys Gln Ser Ser Gly Cys Cys Gln Pro Pro Cys
   65 70 75 80
   Thr Thr Ala Leu Cys Arg Pro Ile Ser Cys Ser Thr Pro Ser Cys Cys
   85 90 95
<210> 63
   <211> 112
   <212> PRT
   <213> Homo sapiens
<400> 63
   Met Cys His Thr Ser His Ser Ser Gly Cys Pro Met Ala Cys Pro Gly
   1 5 10 15
   Ser Pro Cys Cys Val Pro Ser Thr Cys Tyr Pro Pro Glu Gly Tyr Gly
   20 25 30
   Thr Ser Cys Cys Cys Ser Ala Pro Cys Val Ala Leu Leu Cys Arg Pro
   35 40 45
   Leu Cys Gly Val Ser Thr Cys Cys Gln Pro Ala Cys Cys Val Pro Ser
   50 55 60
   Pro Cys Gln Val Ala Cys Cys Val Pro Val Ser Cys Lys Pro Val Leu
   65 70 75 80
   Cys Val Ala Ser Phe Cys Pro Thr Ser Gly Cys Cys Gln Pro Phe Cys
   85 90 95
   Pro Thr Leu Val Tyr Arg Pro Val Thr Trp Ser Thr Pro Thr Gly Cys
   100 105 110
<210> 64
   <211> 172
   <212> PRT
   <213> Homo sapiens
<400> 64
   Met Ser Tyr Asn Cys Cys Ser Gly Asn Phe Ser Ser Arg Ser Cys Gly
   1               5                   10                  15
   Gly Tyr Leu His Tyr Pro Ala Ser Ser Cys Gly Phe Ser Tyr Pro Ser
               20                  25                  30
   Asn Gln Val Tyr Ser Thr Asp Leu Cys Ser Pro Ser Thr Cys Gln Leu
           35                  40                  45
   Gly Ser Ser Leu Tyr Arg Gly Cys Gln Gln Thr Cys Trp Glu Pro Thr
       50                  55                  60
   Ser Cys Gln Thr Ser Tyr Val Glu Ser Ser Pro Cys Gln Thr Ser Cys
   65                  70                  75                  80
   Tyr Arg Pro Arg Thr Ser Leu Leu Cys Ser Pro Cys Gln Thr Thr Tyr
                   85                  90                  95
   Ser Gly Ser Leu Gly Phe Gly Ser Ser Ser Cys Arg Ser Leu Gly Tyr
               100                 105                 110
   Gly Ser Arg Ser Cys Tyr Ser Val Gly Cys Gly Ser Ser Gly Phe Arg
           115                 120                 125
   Ser Leu Gly Tyr Gly Gly Cys Gly Phe Pro Ser Leu Gly Tyr Gly Val
       130                 135                 140
   Gly Phe Cys Arg Pro Thr Tyr Leu Ala Ser Arg Ser Cys Gln Ser Ser
   145                 150                 155                 160
   Cys Tyr Arg Pro Thr Cys Gly Ser Gly Phe Tyr Tyr
                   165                 170
<210> 65
   <211> 105
   <212> PRT
   <213> Homo sapiens
<400> 65
   Met Gly Cys Cys Pro Gly Asp Cys Phe Thr Cys Cys Thr Gln Glu Gln
   1 5 10 15
   Asn Cys Cys Glu Glu Cys Cys Cys Gln Pro Gly Cys Cys Gly Cys Cys
   20 25 30
   Gly Ser Cys Cys Gly Cys Gly Gly Ser Gly Cys Gly Gly Ser Gly Cys
   35 40 45
   Gly Gly Ser Cys Cys Gly Ser Ser Cys Cys Gly Ser Gly Cys Gly Gly
   50 55 60
   Cys Gly Gly Cys Gly Gly Cys Gly Gly Gly Cys Cys Gly Ser Ser Cys
   65 70 75 80
   Cys Gly Ser Ser Cys Cys Gly Ser Gly Cys Cys Gly Pro Val Cys Cys
   85 90 95
   Gln Pro Thr Pro Ile Cys Asp Thr Lys
   100 105
<210> 66
   <211> 88
   <212> PRT
   <213> Homo sapiens
<400> 66
   His Tyr Gly Ser Tyr Tyr Gly Gly Leu Gly Tyr Ser Cys Gly Gly Phe
   1 5 10 15
   Gly Gly Leu Gly Tyr Gly Tyr Gly Cys Gly Cys Gly Ser Phe Cys Arg
   20 25 30
   Arg Gly Ser Gly Cys Gly Tyr Gly Gly Tyr Gly Tyr Gly Ser Gly Phe
   35 40 45
   Gly Ser Tyr Gly Tyr Gly Ser Gly Phe Gly Gly Tyr Gly Tyr Gly Ser
   50 55 60
   Gly Phe Gly Gly Tyr Gly Tyr Gly Cys Cys Arg Pro Ser Tyr Asn Gly
   65 70 75 80
   Gly Tyr Gly Phe Ser Gly Phe Tyr
   85

## Description

Le domaine de l'invention concerne l'utilisation de polypeptides dans des compositions cosmétiques destinées au soin, au renforcement et/ou à la réparation des substrats kératiniques.

L'invention porte notamment sur des polypeptides modifiés de la famille des KAP (Keratin associated protein ou protéine associée aux kératines) et leur utilisation dans des compositions destinées au soin, au traitement, au renforcement et/ou à la réparation des substrats kératiniques, en particulier des fibres kératiniques.
En particulier, l'invention concerne des polypeptides salifiés ou des polypeptides modifiés par liaison covalente de la famille des KAP, adaptés à une meilleure formulation dans les compositions cosmétiques.

Elle porte également sur des procédés de préparation desdits polypeptides modifiés de la famille des KAP, sur des compositions cosmétiques contenant lesdits polypeptides modifiés ainsi que sur des procédés cosmétiques de soin, de renforcement et/ou de réparation des substrats kératiniques mettant en oeuvre lesdites compositions.

Le terme "substrat kératinique" selon l'invention englobe la peau, les ongles et les fibres kératiniques. On entend par "fibres kératiniques", les cheveux, les cils, les sourcils et les poils.

Les kératines sont des composés fondamentaux de la peau, du cheveu, du cil et de l'ongle. Ces protéines fibreuses, insolubles dans l'eau, participent notamment à leur forme, à leur élasticité et à leur résistance.
Dans le cheveu par exemple, il existe deux grands types de protéines :
- les kératines α, divisées notamment en deux groupes -les kératines acides (hHa1-8) et les kératines basiques à neutres (hHb1-6)- sont de longues chaînes polypeptidiques hélicoïdales superenroulées en hélices α qui forment les microfibrilles ou filaments intermédiaires du cortex (FI), insérés dans une matrice de KAPs ;
- les KAP (Keratin Associated Proteins) ou IFAPs (Intermediate Filaments Associated Protein) forment la matrice amorphe entre ces microfibrilles. Il existe 3 types de KAPs réparties en 23 familles à ce jour : les KAP très riches en cystéine (UHS ou Ultra High Sulfur qui contiennent plus de 30 mol% de cystéine), les KAP cystéine) et les KAP riches en glycine et tyrosine (HGT ou High Glycine and Tyrosine).

L'ensemble de ces protéines assure, par le biais de liaisons covalentes (ponts disulfures notamment), liaisons salines, liaisons hydrogène ou liaisons hydrophobes entre les chaînes d'acides aminés, la cohésion de la structure tridimensionnelle du cheveu. En particulier, les séquences des kératines α et des KAPs présentent des motifs répétés, pour les uns riches en résidus lysine, pour les autres en résidus glutamine, qui constituent des substrats potentiels d'enzymes (ex : transglutaminases) aptes à former des ponts interfilaments (FI-FI), inter KAPs (KAP-KAP), ou des laisons covalentes entre les filaments de kératines et les KPAs (FI-KAP), jouant un rôle essentiel dans la structure et la forme de la tige pilaire.

Les substrats kératiniques et les fibres kératiniques en particulier, peuvent être sensibilisés ou affaiblis par l'action chimique d'agents atmosphériques (UV, pollution...), et/ou de traitements (décoloration, coloration, défrisage, permanente...).
On sait par exemple que les cheveux sensibilisés ou fragilisés sont souvent secs, rêches, difficiles à démêler et à coiffer.
On sait aussi que les ongles présentent, de façon fréquente, des défauts de structure et de consistance, qui ont pour effet de rendre la surface des ongles inesthétique, ce qui peut être source de gêne et de désagréments multiples.

On connaît de la demande WO 99/49837 L'OREAL l'utilisation de dérivés de polyamino-acides dans le renforcement et le soin des fibres kératiniques.
La demande WO 03/042387 décrit des séquences nucléotidiques et polypeptidiques de type KAP à activité de liaison à la kératine du cheveu, ainsi que des agents cosmétiques ou thérapeutiques intégrant lesdites séquences.

Mais à la connaissance de la Demanderesse, il n'a jamais été décrit de polypeptides KAP salifiés ou modifiés par liaison covalente adaptés à une meilleure formulation dans des compositions cosmétiques destinées au soin, au traitement, au renforcement et/ou à la réparation des substrats kératiniques, en particulier des fibres kératiniques.

L'invention concerne donc un polypeptide salifié ou modifié par liaison covalente de la famille des KAP, fonctionnalisés pour une meilleure formulation dans une composition cosmétique, c'est-à-dire présentant une bonne solubilité dans les solvants compatibles avec une application topique sur les substrats kératiniques et/ou une bonne affinité et rémanence sur lesdits substrats kératiniques.

Le polypeptide modifié de la famille des KAP selon l'invention est caractérisé en ce qu'il possède la séquence peptidique de n° d'accession SwissProt Q9BYS0 ou un fragment SPCCR de celle-ci et en ce qu'il contient au moins un acide aminé salifié et/ou un acide aminé modifié par liaison covalente.
Ledit fragment (ii) pourra contenir de 3 à 60 acides aminés, en particulier de 3 à 20 acides aminés, et encore plus préférentiellement de 3 à 10 acides aminés et sera en outre caractérisé en ce qu'il possède au moins un acide aminé salifiable et/ou modifiable par liaison covalente, choisi parmi les acides aminés lysine, histidine, arginine, aspartate, glutamate, cystéine, méthionine, tyrosine, thréonine, sérine.

Ce fragment de polypeptide peut être obtenu par protéolyse ou de manière synthétique selon les méthodes connues.

Par 'homologues', on entend selon l'invention toute séquence peptidique identique à au moins 50%, de préférence à au moins 80% et encore plus préférentiellement à au moins 95 % de ladite séquence peptidique ou dudit fragment tels que définis précédemment, chez la même espèce ou chez une espèce différente ; dans ce dernier cas, on le désigne également par 'polypeptide orthologue'.
Et par 'pourcentage d'identité' entre deux séquences peptidiques ou séquences d'acides aminés, on entend désigner un pourcentage de résidus d'acides aminés identiques entre les deux séquences à comparer, obtenu après le meilleur alignement, c'est-à-dire l'alignement optimal réalisé par exemple au moyen de l'algorithme d'homologie locale de Smith et Waterman (1981, Ad. App. Math. 2 :482), au moyen de l'algorithme d'homologie locale de Neddleman et Wunsch (1970, J.Mol. Biol. 48 : 443), au moyen de la méthode de recherche de similarité de Pearson et Lipman (1988, Proc. Natl. Acad. Sci. USA 85 :2444), au moyen de logiciels informatiques utilisant ces algorithmes (GAP, BESTFIT, BLAST P, BLAST N disponible sur le site NCBI, FASTA et TFASTA dans le Wisconsin Genetics Software Package, Genetics Computer Group, 575 Science Dr, Madison, WI).

Les polypeptides KAP originels peuvent être des polypeptides d'origine naturelle ou synthétique.
Par 'origine naturelle', on entend un polypeptide à l'état pur ou en solution à différentes concentrations, obtenu par différents procédés d'extraction à partir d'une matière kératinique (peau, ongle, cheveu, en particulier cheveu) d'origine naturelle.

Par 'origine synthétique', on entend un polypeptide à l'état pur ou en solution à différentes concentrations, obtenu chimiquement ou par production dans un organisme après introduction dans cet organisme des éléments nécessaires à cette production. Par exemple, le polypeptide sera un polypeptide recombinant.

Ces polypeptides de la famille des KAP seront modifiés selon l'invention par un procédé chimique et/ou enzymatique comprenant au moins une réaction choisie parmi une réaction de salification ou une réaction de dérivation (ex : substitution nucléophile ou oxydo-réduction ou encore acylation) comme décrit ci-après.

Par 'acide aminé modifié par liaison covalente' on entend selon l'invention un acide aminé modifié par exemple par une réaction de susbtitution ou une réaction d'oxydoréduction ou encore une réaction d'acylation.

En particulier, le polypeptide modifié de la famille des KAP selon l'invention contient au moins 1 acide aminé salifié et/ou modifié par liaison covalente et jusqu'à 100% d'acides aminés salifiés et/ou modifiés par liaison covalente par rapport au nombre total d'acides aminés salifiables et/ou modifiables par liaison covalente dudit polypeptide natif, de préférence de 20 à 80% et encore plus préférentiellement de 30 à 60% d'acides aminés salifiés et/ou modifiés par liaison covalente par rapport au nombre total d'acides aminés salifiables et/ou modifiables par liaison covalente dudit polypeptide natif.

Par 'acide aminé salifiable' selon l'invention, on peut citer les acides aminés lysine, histidine, arginine, aspartate, et glutamate.

Par 'acide aminé modifiable par liaison covalente' selon l'invention, on peut citer les acides aminés cystéine, méthionine, lysine, histidine, arginine, aspartate, glutamate, tyrosine, thréonine, et sérine.

Selon un premier aspect de l'invention, le polypeptide modifié de la famille des KAP selon l'invention est un polypeptide cationique.
Il peut être obtenu par l'addition d'au moins un acide choisi parmi l'acide chlorhydrique, l'acide acétique, l'acide succinique, l'acide palmitique, l'acide stéarique, l'acide oléique, l'acide béhénique, l'acide 18-méthyl-eicosanoïque, les acides α-hydroxy, les acides β-hydroxy et leurs mélanges.

Selon un autre aspect de l'invention, le polypeptide modifié de la famille des KAP selon l'invention est un polypeptide anionique.
Il peut être obtenu par l'addition d'au moins une base choisie parmi les bases minérales, les dérivés guanidine, les amines I^{aires}, les amines III^{aires} et leurs mélanges.

Selon un autre aspect de l'invention, le polypeptide modifié de la famille des KAP selon l'invention est un polypeptide modifié par liaison covalente chimiquement et/ou enzymatiquement. Il peut être notamment obtenu par l'addition de dérivés électrophiles ou nucléophiles tels qu'un dérivé d'acide carboxylique, un dérivé thiol ou encore un dérivé d'amine.

L'invention concerne également des procédés de préparation dudits polypeptide modifié de la famille des KAP selon l'invention.

Selon un premier aspect, il s'agit d'un procédé de préparation d'un polypeptide salifié de la famille des KAPs comprenant au moins une étape de salification d'un polypeptide KAP en présence d'un acide ou d'une base.
L'acide ou la base réagit spécifiquement avec les chaînes latérales du polypeptide KAP comportant des groupes amines ou acides ionisables (ex : lysine, histidine, arginine, aspartate, glutamate).

Cette réaction est généralement effectuée à une température allant de 0°C à 60°C, de préférence entre 20°C et 45°C réglé à un pH donné variant de 5 à 9 selon la structure et la solubilité du polypeptide de la famille des KAP.
La durée de la réaction peut aller de 15 minutes à 3 heures en fonction de la structure générale dudit polypeptide et du nombre d'acides aminés salifiables présents dans ledit polypeptide.

Selon un mode particulier, la réaction est assistée par solubilisation dans des bains ultrasons selon la technique classique.

De préférence, la réaction sera réalisée dans une base aqueuse, en présence de solvants organiques hydrophiles, tels que des mono-alcools inférieurs, linéaires ou ramifiés, ayant de 1 à 8 atomes de carbone comme l'éthanol, le propanol, le butanol, l'isopropanol, l'isobutanol, des polyéthylèneglycols éventuellement oxyéthylénés, des polyols tels que le propylèneglycol, l'isoprèneglycol, le butylène glycol, le glycérol, le sorbitol et ses dérivés et les éthers de propylène glycol.

Mais elle peut également être réalisée dans ou en présence d'une base huileuse comprenant des huiles telles que les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

La réaction peut également être réalisée dans une base émulsionnée à partir d'une phase aqueuse et d'une phase huileuse.

Les polypeptides salifiés obtenus peuvent être utilisés tels quels, sans étape de purification supplémentaire.

En particulier, le procédé comprendra les étapes suivantes:
- on fait réagir, dans une base aqueuse ou huileuse ou émulsionnée, (a) un polypeptide de la famille des KAP de séquence peptidique de n° d'accession SwissProt Q9BYS0 ou un fragment SPCCR de celle-ci et en ce qu'il contient au moins un acide aminé salifié et/ou un acide aminé modifié par liaison covalente - on ajuste la température de 20°C à 40°C et le pH de 5 à 9 ; et
- on poursuit la réaction pendant une durée allant de 15 minutes à 3 heures.

Pour l'obtention d'un polypeptide cationique, on utilisera un acide choisi de préférence parmi l'acide chlorhydrique, l'acide acétique, l'acide succinique, l'acide palmitique, l'acide stéarique, l'acide oléique, l'acide béhénique, l'acide 18-méthyl-eicosanoïque, les acides α-hydroxy (ex : acide glycolique, acide lactique, acide citrique), les acides β-hydroxy (ex : acide salicylique) et leurs mélanges.

Pour l'obtention d'un polypeptide cationique adapté à une formulation aqueuse, on utilisera préférentiellement l'acide chlorhydrique, l'acide acétique, l'acide succinique, les acides α-hydroxy, ou les acides β-hydroxy comme l'acide citrique , ou leurs mélanges. Pour l'obtention d'un polypeptide cationique adapté à une formulation huileuse, on utilisera préférentiellement l'acide palmitique, l'acide stéarique, l'acide oléïque, l'acide béhénique, ou l'acide 18-méthyl-éicosanoïque, ou leurs mélanges.

Pour l'obtention d'un polypeptide anionique, on utilisera une base choisie de préférence parmi les bases minérales (ex : soude, potasse, bicarbonate), les bases organiques (ex : ammoniaque), les dérivés guanidine (ex : hydroxyde de guanidinium, tétraméthyl-guanidine), les amines I^{aires} (ex : glucamine) et III^{aires} (ex : tri-éthanolamine, N,N-diéthyl-éthanolamine, N-méthyl - diéthanolamine) et leurs mélanges.

Un autre aspect de l'invention concerne un procédé de préparation chimique et/ou enzymatique d'un polypeptide modifié par liaison covalente de la famille des KAPs comprenant au moins une étape de dérivation d'un polypeptide de la famille des KAP.

La réaction de dérivation consiste par exemple en une modification avec formation de liaison covalente de tout groupement fonctionnel réactif situé sur les chaînes latérales (NH2, COOH, OH, SH, S-CH3, résidu arginyl, résidu histidyl).
En particulier, on cherchera à modifier les acides aminés thiolés du polypeptide KAP (ex : cystéine, méthionine), les acides aminés cationiques (ex : lysine, histidine, arginine), les acides aminés anioniques (ex : aspartate, glutamate), et/ou les acides aminés à fonction hydroxyle (ex : tyrosine, thréonine, sérine).
Et encore plus préférentiellement, on privilégiera les modifications de la cystéine, la lysine, l'histidine, l'arginine, la sérine et la tyrosine.

Ces modifications par réaction de dérivation sont bien connues de l'homme du métier ; elles sont notamment décrites dans « Techniques in protein modification » de R.L. Lundblad, p. 63 à 268, 1994 CRC Press, dans « Chemistry of the Amino acids » de J.P. Greenstein et M. Winitz, vol. 2, p. 883 à 1268, Krieger Publishing Company , reprint edition 1984 ou encore dans « The Chemical Synthesis of Peptides » de J. Jones, p. 17 à 94, Oxford Science Publications, 1991.

A titre d'exemple de réactions de dérivation, on peut notamment citer les réactions de :
a) substitution ou d'acylation par les fonctions nucléophiles des KAP (ex : cystéine, lysine, tyrosine, histidine, sérine) sur tout électrophile et en particulier sur les dérivés d'acide carboxylique (ex : esters, anhydride, halogénures dont chlorures et imidazolides) et les dérivés d'acide sulfonique (ex : chlorures) ;
b) oxydation (si SH libre) ou réduction (si SS) de cystéines de polypeptides KAP, par exemple par réaction avec un dérivé thiol ou par tout autre réducteur ou oxydant.

Ces réactions s'effectuent généralement à une température allant de la température ambiante à la température d'ébullition du solvant de réaction.
En particulier, on pourra utiliser une réaction à une température allant de 20 à 180°C, de préférence de 20 à 80°C. On pourra utiliser un chauffage classique ou un chauffage par une énergie micro-ondes.
La durée de la réaction est généralement de 30 minutes à 8 heures en fonction de la structure dudit polypeptide (taille, solubilité et nombre d'acides aminés substituables).
La réaction pourra être réalisée dans une base aqueuse, une base huileuse, une base émulsionnée telles que décrites précédemment pour la réaction de salification, ou encore dans un milieu sans solvant.

Par exemple, le procédé de préparation d'un polypeptide modifié par liaison covalente de la famille des KAPs par une réaction de substitution ou d'acylation comprendra les étapes suivantes :
- on fait réagir, dans une base aqueuse, huileuse ou émulsionnée ou encore dans un milieu sans solvant, (a) un polypeptide de la famille des KAP possédant la séquence peptidique de n° d'accession SwissProt Q9BYS0 ou un fragment SPCCR de celle-ci et en ce qu'il contient au moins un acide aminé salifié et/ou un acide aminé modifié par liaison covalente - on ajuste la température de 20°C à 180°C ;
- on poursuit la réaction pendant une durée allant de 30 minutes à 8 heures.

De façon avantageuse, la réaction de substitution ou d'acylation pourra être catalysée en présence d'une base. De préférence, on utilisera une amine III^{aire} (ex : tri-éthanolamine, N,N-diéthyl-éthanolamine, N-méthyl-diéthanolamine).

Pour l'obtention d'un polypeptide KAP modifié par liaison covalente adapté à une solubilisation dans les solvants aqueux, on utilisera par exemple un dérivé anhydride (ex : anhydride succinique).
Pour l'obtention d'un polypeptide KAP modifié par liaison covalente adapté à une solubilisation dans les huiles, on utilisera par exemple un chlorure d'acide gras (ex : chlorure d'acide oléique).

Selon un autre mode de réalisation, le procédé de préparation d'un polypeptide modifié par liaison covalente de la famille des KAPs par une réaction d'oxydo-réduction comprendra les étapes suivantes :
- on fait réagir, dans une base aqueuse ou huileuse ou émulsionnée ou dans un milieu sans solvant, (a) un polypeptide de la famille des KAP possédant la séquence peptidique de n° d'accession SwissProt Q9BYS0 ou un fragment SPCCR de celle-ci et en ce qu'il contient au moins un acide aminé salifié et/ou un acide aminé modifié par liaison covalente - on ajuste la température de 20°C à 80°C ;
- on poursuit la réaction pendant une durée allant de 30 minutes à 8 heures.

Pour l'obtention d'un polypeptide KAP modifié par liaison covalente adapté à une solubilisation dans les solvants aqueux, on utilisera par exemple l'acide thioglycolique. Pour l'obtention d'un polypeptide KAP modifié par liaison covalente adapté à une solubilisation dans les huiles, on utilisera par exemple un octadécane thiol.

Le polypeptide modifié de la famille des KAP selon l'invention présentent une bonne solubilité dans les solvants aqueux et/ou alcooliques et/ou huileux, c'est-à-dire une solubilité au moins égale à celle des polypeptides de la famille des KAP originels (non modifiés), et de préférence une solubilité supérieure, par exemple supérieure de 10 à 15% par rapport à la solubilité des polypeptides de la famille des KAP non modifiés.
Par "solubilité" dans les solvants, on entend selon l'invention la capacité d'un polypeptide, mélangé dans un ou plusieurs solvants, à se dissoudre dans le(s)dit(s) solvant(s).
Cette solubilité peut être évaluée selon les techniques connues de l'homme du métier.
Par exemple, le principe peut consister à mélanger ledit polypeptide et un solvant aqueux et/ou alcoolique et/ou huileux, et à réaliser une observation macroscopique après un temps donné. On mesure ainsi, par exemple au bout d'une semaine, la présence ou non de cristaux et/ou de dépôt dans la solution, ce qui permet de définir pour chaque polypeptide, le solvant aqueux et/ou alcoolique et/ou huileux le plus adapté à une dissolution complète dudit polypeptide.

Les polypeptides modifiés de la famille des KAP selon l'invention présentent également une bonne affinité et une bonne rémanence sur le substrat kératinique, en particulier sur la fibre kératinique, voire une affinité et une rémanence supérieure, par exemple supérieure de 10 à 15% par rapport aux polypeptides de la famille des KAP originels (non modifiés).
Par "affinité" sur le substrat kératinique, on entend selon l'invention la capacité d'un polypeptide à se lier sur le substrat kératinique au moment de l'application de la composition sur ledit substrat kératinique.
Par "rémanence" sur le substrat kératinique, on entend selon l'invention la capacité d'un polypeptide à rester lié au substrat kératinique après application de la composition sur ledit substrat kératinique, en particulier après un ou plusieurs lavages ou shampooings.

L'affinité et la rémanence dudit polypeptide sur la fibre kératinique peuvent être mesurées classiquement par dosage UV (ex : spectrophotométrie de masse), qui permet de définir la quantité de polypeptide lié ou fixé sur la fibre kératinique.
Le principe peut consister par exemple à déposer ledit polypeptide ou une composition contenant ledit polypeptide sur une mèche, à laisser agir pendant un temps donné, puis à rincer la mèche, la laver avec un shampooing, la rincer à nouveau et la sécher sous sèche-cheveux.
Des portions de cheveux différents sont coupés au milieu du cheveu et introduits dans un spectrophotomètre de masse. Les résultats sont exprimés par un rapport de la masse de polypeptide fixé sur le cheveu sur la masse de cheveu. On peut ainsi mesurer le pourcentage de polypeptide fixé sur ladite mèche au moment du dépôt (affinité), ou après un nombre de shampooings donnés (rémanence).
L'affinité et la rémanence peut également être mesurée classiquement par microscopie optique, au moment du dépôt et après un nombre de shampooings donnés, par exemple après 5 shampooings. On évalue ainsi qualitativement le dépôt sur ladite mèche.

L'invention concerne également l'utilisation cosmétique d'au moins un polypeptide modifié de la famille des KAP tel que défini selon l'invention, dans une composition cosmétique comprenant un milieu physiologiquement acceptable.

Par "milieu physiologiquement acceptable", on entend selon l'invention un milieu cosmétiquement acceptable et compatible avec les substrats kératiniques.
D'une manière générale, un milieu compatible avec les substrats kératiniques peut être anhydre ou aqueux ; il peut ainsi comprendre une phase aqueuse et/ou une phase grasse, comprenant au moins un corps gras, choisi parmi les huiles, les cires, les corps gras pâteux et leurs mélanges.

Par phase aqueuse, on entend de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols, ou bien encore des éthers en C₂ et des aldéhydes en C₂-C₄ hydrophiles. L'eau ou le mélange d'eau et de solvants organiques hydrophiles peut être présent dans la composition selon l'invention en une teneur allant de 0,1 % à 99 % en poids, par rapport au poids total de la composition, et de préférence de 10 % à 80 % en poids.

Par phase grasse, on entend une phase comprenant au moins un corps gras, choisi parmi les huiles, les cires, les corps gras pâteux et leurs mélanges.

En particulier, ledit polypeptide modifié de la famille des KAP est destiné à :
- maintenir et/ou restaurer et/ou améliorer le contenu protéique du substrat kératinique ;
- maintenir et/ou restaurer et/ou améliorer l'état de surface desdites substrats kératiniques ;
- conserver et/ou restaurer et/ou améliorer les propriétés physiques et/ou mécaniques des substrats kératiniques.

Il sera ainsi notamment destiné à améliorer la douceur des fibres kératiniques, et/ou leur souplesse et/ou leur élasticité et/ou leur résistance à la casse, et/ou leur volume et/ou leur maintien à la coiffure, et/ou leur résistance aux traitements chimiques.

Le polypeptide modifié de la famille des KAP selon l'invention est présent dans la composition en une quantité suffisante en fonction de l'effet recherché. En particulier cette quantité pourra aller de 0,001 à 30% en poids par rapport au poids total de la composition, de préférence de 0,01 à 15% et encore plus préférentiellement de 0,1 à 5% en poids par rapport au poids total de la composition.

L'invention porte également sur une composition comprenant, dans un milieu physiologiquement acceptable adapté à une application topique sur les substrats kératiniques, au moins un polypeptide modifié de la famille des KAP tel que défini précédemment.

Lorsque le milieu de la composition comprend majoritairement des solvants aqueux, on utilisera préférentiellement au moins un polypeptide modifié de la famille des KAP susceptible d'être obtenu par un procédé chimique comprenant au moins une étape de salification par addition d'au moins un acide choisi parmi l'acide chlorhydrique, l'acide acétique, l'acide citrique, l'acide succinique, et leurs mélanges.

Lorsque le milieu de la composition comprend majoritairement des solvants huileux, on utilisera préférentiellement au moins un polypeptide modifié de la famille des KAP susceptible d'être obtenu par un procédé chimique comprenant au moins une étape de salification par addition d'au moins un acide choisi parmi l'acide palmitique, l'acide stéarique, l'acide oléique, l'acide béhéniquen l'acide 18-methyl-éicosanoïque et leurs mélanges.

En particulier, ledit polypeptide modifié de la famille des KAP est présent dans la composition en une quantité allant de 0,001 à 30% en poids par rapport au poids total de la composition, de préférence de 0,01 à 15% et encore plus préférentiellement de 0,1 à 5% en poids par rapport au poids total de la composition.

La composition selon l'invention peut comprendre en outre au moins un ingrédient cosmétique, associé audit polypeptide modifié de la famille des KAP, de façon covalente ou non covalente.

Cet ingrédient cosmétique peut être tout ingrédient connu dans le domaine cosmétique.

En particulier, l'ingrédient cosmétique peut être choisi parmi un agent de conditionnement, un agent de soin et/ou de traitement des substrats kératiniques, un adjuvant cosmétique ou agent de formulation et leurs mélanges.

Les agents de conditionnement, de soin et/ou de traitement des substrats kératiniques sont notamment destinés à améliorer l'aspect et/ou l'état de surface des substrats kératiniques (ex : cheveux plus doux, plus lisses, moins fourchus, ongles plus brillants...), à préserver et/ou améliorer les propriétés physiques et/ou mécaniques des substrats kératiniques (ex : cheveux plus forts, plus faciles à coiffer, ongles moins cassants...), en particulier lorsque les substrats kératiniques sont sensibilisés ou affaiblis par l'action chimique d'agents atmosphériques et/ou de traitements.

Comme agents de conditionnement des substrats kératiniques, on peut citer par exemple les huiles, les cires, les céramides, les gommes, les silicones, les polysiloxanes, les polymères, les polymères filmogènes, les polymères fixants et leurs mélanges

Comme agents de soin et/ou de traitement des substrats kératiniques, on peut citer notamment les agents hydratants, les agents stimulant la synthèse de macromolécules dermiques ou épidermiques et/ou empêchant leur dégradation, les agents anti-pollution ou antiradicalaires, les agents adoucissants, les vitamines, les acides aminés, les protéines, les agents anti-pelliculaires, les agents anti-séborrhéiques, les agents réducteurs, les précurseurs de colorants capillaires, les colorants capillaires directs, les agents oxydants, les agents favorisant le recourbement des cils, les agents favorisant la pousse des ongles, les agents durcisseurs d'ongles...

Comme adjuvants cosmétiques ou agents de formulation utilisés classiquement dans les compositions cosmétiques, on peut citer les solvants organiques, les agents tensioactifs, les agents plastifiants, les agents épaississants, les agents émulsionnants, les conservateurs, les filtres UV, les charges, les matières colorantes, les parfums, les agents opacifiants, les agents humectants, les agents d'ajustement et de fixation du pH, les agents anti-bactériens, les agents antifongiques, les agents propulseurs et leurs mélanges.

L'homme du métier adaptera la quantité d'ingrédient cosmétique dans la composition en fonction de l'effet recherché et de façon telle que la quantité d'ingrédient cosmétique présente dans la composition n'altère pas les propriétés recherchées du polypeptide modifié de la famille des KAP.

En particulier, ledit ingrédient cosmétique sera présent dans la composition en une quantité pouvant aller de 0,001 à 20% en poids par rapport au poids total de la composition, de préférence de 0,01 à 10% et encore plus préférentiellement de 0,1 à 2% en poids par rapport au poids total de la composition.

Les compositions selon l'invention comprenant, dans un milieu physiologiquement acceptable, au moins un polypeptide modifié de la famille des KAP, pourront se présenter sous toutes les formes galéniques connues adaptées à une application topique sur les substrats kératiniques (peau, cils, ongles, cheveux).
L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les compositions selon l'invention adaptées à une application topique peuvent se présenter notamment sous forme de solution aqueuse, hydroalcoolique ou huileuse, d'émulsions huile-dans-eau (H/E) ou eau-dans-huile (E/H) ou multiple (E/H/E ou H/E/H), de gel aqueux ou huileux, de produits anhydres déshydratés, ou de dispersions d'une phase huileuse dans une phase aqueuse à l'aide de sphérules, ces sphérules pouvant être des nanoparticules polymériques telles que des nanosphères et les nanocapsules, ou des vésicules lipidiques de type ionique (liposomes) et/ou non ionique.

Lorsque la composition utilisée selon l'invention est une émulsion, la proportion de la phase grasse peut aller de 5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré. L'émulsionnant et le coémulsionnant sont présents, dans la composition, en une proportion allant de 0,3 à 30 % en poids, et de préférence de 0,5 à 20 % en poids par rapport au poids total de la composition.

Pour une application sur les cheveux en particulier, la composition pourra se présenter sous la forme de crèmes, de lotions, de gels, d'émulsions, de mousses ou sous la forme de compositions pour aérosol comprenant également un agent propulseur sous pression. Elle peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant, mousse), d'un shampoing colorant, d'une composition de permanente, d'un masque traitant, d'une crème ou d'un gel moussant de nettoyage des cheveux. Elle peut encore se présenter sous forme de teinture ou de mascara capillaire à appliquer au pinceau ou au peigne.

Pour une application sur les cils, la composition se présentera de préférence sous la forme d'un mascara pouvant être une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

Pour une application sur les ongles, la composition pourra être un produit de maquillage des ongles tel qu'un vernis à ongles coloré, une base pour les ongles ou "base-coat" en terminologie anglo-saxonne, une composition de finition, encore appelée "top-coat", à appliquer sous ou sur le produit de maquillage des ongles, un dissolvant pour vernis à ongles ou bien encore un produit de soin cosmétique des ongles tel qu'une base traitante destinée à protéger, à renforcer et/ou réparer les ongles.

Pour une application sur la peau, la composition pourra être plus ou moins fluide et avoir l'aspect d'une crème blanche ou colorée, d'une pommade, d'un lait, d'une lotion, d'un sérum, d'une pâte, d'une mousse. Elle peut également se présenter sous forme solide, en particulier sous forme de stick. Elle peut être utilisée comme produit de soin et/ou comme produit de maquillage pour la peau.

L'application desdites compositions sera réalisée par tout moyen adéquat, tel qu'un pinceau, une brosse, un spray ou les doigts par exemple.

L'invention concerne également un procédé cosmétique de soin, de traitement, de renforcement et/ou de réparation des substrats kératiniques, caractérisé en ce que l'on applique sur la peau, les cheveux, les cils ou les ongles une composition telle que définie précédemment, suivi éventuellement d'un rinçage.

Par 'soin des substrats kératiniques', on entend selon l'invention une composition destinée à améliorer l'aspect et/ou l'état de surface des substrats kératiniques. Le soin des substrats kératiniques pourra consister à rendre les cheveux plus doux, plus lisses, moins fourchus ; et les ongles moins cassants, moins dédoublés.

Par 'renforcement et/ou réparation des substrats kératiniques' selon l'invention, on entend une composition destinée à conserver et/ou restaurer et/ou améliorer les propriétés physiques et/ou mécaniques des substrats kératiniques, pouvant se manifester par exemple:
- soit par une rigidification des substrats kératiniques, qui leur procure davantage de consistance et de corps, ainsi qu'un toucher plus agréable. Il en résulte par exemple une augmentation du volume des fibres kératiniques ainsi qu'une facilité et un maintien du coiffage plus élevés ;
- soit par une meilleure élasticité et/ou une meilleure résistance à des forces mécaniques de traction qui leur sont appliquées, par exemple lors d'un peignage pour les fibres kératiniques, en particulier sur des cheveux africains ou tous cheveux fragilisés ou sensibilisés.

Les cheveux sont ainsi plus forts, plus souples, plus doux, plus lisses et plus faciles à coiffer. Les ongles sont plus lisses, plus brillants, moins abîmés, moins dédoublés et moins cassants.

Selon un premier mode, la composition pourra être appliquée en pré-traitement d'un traitement susceptible de sensibiliser les substrats kératiniques (ex : avant une coloration d'oxydation).

Selon un autre mode de réalisation, la composition pourra êre appliquée en post-traitement d'un traitement susceptible de sensibiliser les substrats kératiniques (ex : après un traitement de peeling).

La composition contenant le polypeptide modifié de la famille des KAP pourra également constituer la composition de traitement (associé à un colorant).

L'application des compositions selon le procédé de l'invention se fera selon la technique d'utilisation habituelle desdites compositions. Par exemple :
- l'application sur cheveux peut avoir lieu avant (lotion, 1 heure avant), pendant (shampooing) ou après (lotions sprays) le shampooing ; la composition peut être appliquée sur cheveux secs (laques, sprays, lotions) ou sur cheveux humides (compositions de déformation permanente ou de mise en plis) ;
- l'application d'une base de soin sur les ongles pourra se faire avant ou après la pose du vernis ;
- l'application d'une composition de soin pour cils pourra être appliquée juste après application du mascara ;
- l'application d'une composition de soin pour la peau pourra être appliquée juste après une solution de nettoyage.

En particulier, le procédé selon l'invention sera destiné au soin, au traitement, au renforcement et/ou à la réparation des substrats kératiniques sensibilisées, et préférentiellement des cheveux sensibilisés.
Par 'cheveux sensibilisés', on entend des cheveux fragilisés ou abîmés par des traitements capillaires comme des défrisants, des permanentes ou des colorations, ou par l'effet d'agents atmosphériques. Les cheveux sensibilisés sont rèches après le lavage et le séchage, avec une moindre tenue mécanique, davantage d'électricité statique et un aspect terne.

Les compositions peuvent être appliquées quotidiennement ou à une fréquence de 2 à 3 applications par semaine, ceci pendant une durée de 1 à 3 mois, renouvelable selon le degré d'altération des substrats kératiniques de l'individu à traiter.

Les compositions selon l'invention seront ainsi destinées à améliorer l'aspect et/ou l'état de surface des substrats kératiniques, en particulier à rendre les cheveux plus doux, plus lisses, moins fourchus, plus résistants à la casse, à leur donner plus de consistance et de corps, notamment plus de volume et plus de maintien pour la coiffure, plus d'élasticité et de souplesse, ainsi qu'une meilleure résistance aux traitements susceptibles de les fragiliser (ex : coloration, permanente).

L'invention est illustrée plus en détail dans l'exemple non limitatif suivant :

### EXEMPLE : Préparation d'un Polypeptide KAP modifié de type cationique

On choisit comme séquence native le fragment SPCCR de la séquence SEQ ID N°9 (n° d'accession Q9BYS0) correspondant à un fragment de séquence répétée SZCCX (Z : P et X : R) contenant un acide aminé arginine (salifiable) et deux cystéine.
Ce fragment de séquence peut être obtenu par voie de synthèse chimique selon les techniques classiques.

On réalise ensuite une réaction de salification qui consiste à :
- mettre ledit fragment de séquence à réagir dans une base aqueuse en présence d'un mélange éthanol / propylène glycol et d'acide chlorhydrique ;
- ajuster la température à 40°C et le pH à 5 au minimum ;
- poursuivre la réaction pendant une durée suffisante pour salifier l'arginine présente dans la séquence, par exemple 1 heure environ.

Le polypeptide cationique obtenu est extrait de ladite solution de salification ; il peut être utilisé tel quel ou subir une étape de purification supplémentaire.
Ce polypeptide est particulièrement adapté à une incorporation dans une formulation cosmétique de type aqueuse.

Selon une alternative visant à obtenir un polypeptide particulièrement adapté à une incorporation dans une formulation cosmétique de type huileuse, on utilise une base émulsionnée et l'on remplace l'acide chlorhydrique par l'acide oléique dans le procédé décrit précédemment.

## Revendications

1. Polypeptide modifié de la famille des KAP **caractérisé en ce qu'**il possède la séquence peptidique de n° d'accession SwissProt Q9BYS0 ou un fragment SPCCR de celle-ci et **en ce qu'**il contient au moins un acide aminé salifié et/ou un acide aminé modifié par liaison covalente.

2. Polypeptide modifié selon la revendication 1, **caractérisé en ce que** ledit fragment contient de 3 à 60 acides aminés, en particulier de 3 à 20 acides aminés et encore plus préférentiellement de 3 à 10 acides aminés.

3. Polypeptide modifié selon l'une quelconque des revendications 1 à 2, **caractérisé en ce qu'**il contient de 1 à 100% d'acides aminés salifiés et/ou modifiés par liaison covalente, de préférence de 20 à 80% et encore plus préférentiellement de 30 à 60% d'acides aminés salifiés et/ou modifiés par liaison covalente par rapport au nombre total d'acides aminés salifiables et/ou modifiables par liaison covalente dudit polypeptide natif.

4. Polypeptide modifié selon l'une quelconque des revendication 1 à 3, **caractérisé en ce que** lesdits acides aminés salifiables et/ou modifiables par liaison covalente sont choisis parmi les acides aminés lysine, histidine, arginine, aspartate, glutamate, cystéine, méthionine, tyrosine, thréonine, et sérine.

5. Polypeptide modifié selon l'une quelconque des revendications 1 à 4, **caractérisé en ce qu'**il s'agit d'un polypeptide cationique.

6. Polypeptide modifié selon l'une quelconque des revendications 1 à 4, **caractérisé en ce qu'**il s'agit d'un polypeptide anionique.

7. Procédé de préparation d'un polypeptide salifié de la famille des KAPs comprenant les étapes suivantes :
- on fait réagir, dans une base aqueuse ou huileuse ou émulsionnée, (a) un polypeptide de la famille des KAP possédant la séquence peptidique de n° d'accession SwissProt Q9BYS0 ou un fragment SPCCR de celle-ci ; et (b) au moins composé choisi parmi un acide ou une base;
- on ajuste la température de 20°C à 40°C et le pH de 5 à 9 ; et
- on poursuit la réaction pendant une durée allant de 15 minutes à 3 heures.

8. Procédé selon la revendication 7, **caractérisé en ce que** l'acide est choisi parmi l'acide chlorhydrique, l'acide acétique, l'acide succinique, l'acide palmitique, l'acide stéarique, l'acide oléique, l'acide béhénique, l'acide 18-méthyleicosanoïque, les acides α-hydroxy, les acides β-hydroxy et leurs mélanges.

9. Procédé selon la revendication 7, **caractérisé en ce que** la base est choisie parmi les bases minérales, les bases organiques, les dérivés guanidine, les amines I^{aires} , les amines III^{aires} et leurs mélanges.

10. Procédé de préparation d'un polypeptide modifié par liaison covalente de la famille des KAPs par une réaction de substitution ou d'acylation comprenant les étapes suivantes :
- on fait réagir, dans une base aqueuse ou huileuse ou émulsionnée ou dans un milieu sans solvant (a) un polypeptide de la famille des KAP possédant possédant la séquence peptidique de n° d'accession SwissProt Q9BYS0 ou un fragment SPCCR de celle-ci; et (b) au moins un dérivé d'acide carboxylique ;
- on ajuste la température de 20°C à 180°C ;
- on poursuit la réaction pendant une durée allant de 30 minutes à 8 heures.

11. Procédé de préparation d'un polypeptide modifié par liaison covalente de la famille des KAPs par une réaction d'oxydo-réduction comprenant les étapes suivantes :
- on fait réagir, dans une base aqueuse ou huileuse ou émulsionnée ou dans un milieu sans solvant, (a) un polypeptide de la famille des KAP possédant la séquence peptidique de n° d'accession SwissProt Q9BYS0 ou un fragment SPCCR de celle-ci; et (b) au moins un dérivé thiol;
- on ajuste la température de 20°C à 80°C ;
- on poursuit la réaction pendant une durée allant de 30 minutes à 8 heures.

12. Utilisation d'au moins un polypeptide modifié de la famille des KAPs tel que défini dans l'une quelconque des revendications 1 à 6 dans une composition cosmétique comprenant un milieu physiologiquement acceptable.

13. Utilisation d'au moins un polypeptide modifié de la famille des KAPs tel que défini dans l'une quelconque des revendications 1 à 6 dans une composition cosmétique, comme agent destiné à maintenir et/ou restaurer et/ou améliorer le contenu protéique du substrat kératinique.

14. Utilisation selon la revendication 12, **caractérisée en ce que** ledit polypeptide modifié de la famille des KAP est destiné à conserver et/ou restaurer et/ou améliorer les propriétés physiques et/ou mécaniques des substrats kératiniques.

15. Utilisation selon la revendication 12, **caractérisée en ce que** ledit polypeptide modifié de la famille des KAP est destiné à maintenir et/ou restaurer et/ou améliorer l'état de surface desdites substrats kératiniques.

16. Utilisation selon l'une quelconque des revendications 12 à 15, **caractérisée en ce que** ledit polypeptide modifié de la famille des KAP est destiné à améliorer la douceur des fibres kératiniques, et/ou leur souplesse et/ou leur élasticité et/ou leur résistance à la casse, et/ou leur volume et/ou leur maintien à la coiffure, et/ou leur résistance aux traitements chimiques.

17. Utilisation selon l'une quelconque des revendications 12 à 16, **caractérisée en ce que** ledit polypeptide modifié de la famille des KAP est présent dans la composition en une quantité allant de 0,001 à 30% en poids par rapport au poids total de la composition, de préférence de 0,01 à 15% et encore plus préférentiellement de 0,1 à 5% en poids par rapport au poids total de la composition.

18. Composition comprenant, dans un milieu physiologiquement acceptable adapté à une application topique sur les substrats kératiniques, au moins un polypeptide modifié de la famille des KAP tel que défini dans l'une quelconque des revendications 1 à 6.

19. Composition selon la revendication 18, **caractérisée en ce que** ledit polypeptide modifié de la famille des KAP est présent dans la composition en une quantité allant de 0,001 à 30% en poids par rapport au poids total de la composition, de préférence de 0,01 à 15% et encore plus préférentiellement de 0,1 à 5% en poids par rapport au poids total de la composition.

20. Procédé cosmétique comprenant le soin, le traitement, le renforcement et/ou la réparation des substrats kératiniques, **caractérisé en ce que** l'on applique sur la peau, les cheveux, les cils ou les ongles une composition telle que définie dans l'une des revendications 18 ou 19.

21. Procédé selon la revendication 20, **caractérisé en ce que** la composition est appliquée sur des substrats kératiniques sensibilisés, en particulier des fibres kératiniques sensibilisées.

## Patentansprüche

1. Modifiziertes Polypeptid aus der KAP-Familie, **dadurch gekennzeichnet, dass** es die Peptidsequenz der SwissProt-Zugangsnr. Q9BYS0 oder ein Fragment SPCCR von dieser besitzt und dass es mindestens eine versalzte Aminosäure und/oder eine durch kovalente Bindung modifizierte Aminosäure enthält.

2. Modifiziertes Polypeptid nach Anspruch 1, **dadurch gekennzeichnet, dass** das Fragment 3 bis 60 Aminosäuren, insbesondere 3 bis 20 Aminosäuren und noch stärker bevorzugt 3 bis 10 Aminosäuren enthält.

3. Modifiziertes Polypeptid nach einem der Ansprüche 1 bis 2, **dadurch gekennzeichnet, dass** es 1 bis 100% versalzte und/oder durch kovalente Bindung modifizierte Aminosäuren, vorzugsweise 20 bis 80% und noch stärker bevorzugt 30 bis 60% versalzte und/oder durch kovalente Bindung modifizierte Aminosäuren, bezogen auf die gesamte Anzahl an versalzbaren und/oder durch kovalente Bindung modifizierbaren Aminosäuren des nativen Polypeptids, enthält.

4. Modifiziertes Polypeptid nach einem der Ansprüche 1 bis 3, **dadurch gekennzeichnet, dass** die versalzbaren und/oder durch kovalente Bindung modifizierbaren Aminosäuren aus den Aminosäuren Lysin, Histidin, Arginin, Aspartat, Glutamat, Cystein, Methionin, Tyrosin, Threonin und Serin ausgewählt sind.

5. Modifiziertes Polypeptid nach einem der Ansprüche 1 bis 4, **dadurch gekennzeichnet, dass** es sich um ein kationisches Polypeptid handelt.

6. Modifiziertes Polypeptid nach einem der Ansprüche 1 bis 4, **dadurch gekennzeichnet, dass** es sich um ein anionisches Polypeptid handelt.

7. Verfahren zur Herstellung eines versalzten Polypeptids aus der KAP-Familie, wobei man in den folgenden Schritten:
- in einer wässrigen oder öligen oder emulgierten Base: (a) ein Polypeptid aus der KAP-Familie, das die Peptidsequenz der SwissProt-Zugangsnr. Q9BYS0 oder ein Fragment SPCCR von dieser besitzt, und (b) mindestens aus einer Säure oder einer Base ausgewählte Verbindung umsetzt,
- die Temperatur von 20°C bis 40°C und den pH von 5 bis 9 einstellt und
- die Reaktion während einer Dauer von 15 Minuten bis 3 Stunden verfolgt.

8. Verfahren nach Anspruch 7, **dadurch gekennzeichnet, dass** die Säure aus Salzsäure, Essigsäure, Bernsteinsäure, Palmitinsäure, Stearinsäure, Ölsäure, Behensäure, 18-Methyleicosansäure, α-Hydroxysäuren, β-Hydroxysäuren und deren Gemischen ausgewählt wird.

9. Verfahren nach Anspruch 7, **dadurch gekennzeichnet, dass** die Base aus mineralischen Basen, organischen Basen, Guanidinderivaten, primären Aminen, tertiären Aminen und deren Gemischen ausgewählt wird.

10. Verfahren zur Herstellung eines durch kovalente Bindung modifizierten Polypeptids aus der KAP-Familie durch eine Substitutions- oder Acylierungsreaktion, wobei man in den folgenden Schritten:
- in einer wässrigen oder öligen oder emulgierten Base oder in einem Lösungsmittel-freien Medium: (a) ein Polypeptid aus der KAP-Familie, das die Peptidsequenz der SwissProt-Zugangsnr. Q9BYS0 oder ein Fragment SPCCR von dieser besitzt, und (b) mindestens ein Carbonsäurederivat umsetzt,
- die Temperatur von 20°C bis 180°C einstellt und
- die Reaktion während einer Dauer von 30 Minuten bis 8 Stunden verfolgt.

11. Verfahren zur Herstellung eines durch kovalente Bindung modifizierten Polypeptids aus der KAP-Familie durch eine Redoxreaktion, wobei man in den folgenden Schritten:
- in einer wässrigen oder öligen oder emulgierten Base oder in einem Lösungsmittel-freien Medium: (a) ein Polypeptid aus der KAP-Familie, das die Peptidsequenz der SwissProt-Zugangsnr. Q9BYS0 oder ein Fragment SPCCR von dieser besitzt, und (b) mindestens ein Thiolderivat umsetzt,
- die Temperatur von 20°C bis 80°C einstellt und
- die Reaktion während einer Dauer von 30 Minuten bis 8 Stunden verfolgt.

12. Verwendung mindestens eines modifizierten Polypeptids aus der KAP-Familie nach einem der Ansprüche 1 bis 6 in einer kosmetischen Zusammensetzung, die ein physiologisch verträgliches Medium umfasst.

13. Verwendung mindestens eines modifizierten Polypeptids aus der KAP-Familie nach einem der Ansprüche 1 bis 6 in einer kosmetischen Zusammensetzung als Mittel, das dazu dient, den Proteingehalt des Keratinsubstrats aufrechtzuerhalten und/oder wiederherzustellen und/oder zu verbessern.

14. Verwendung nach Anspruch 12, **dadurch gekennzeichnet, dass** das modifizierte Polypeptid aus der KAP-Familie dazu dient, die physikalischen und/oder mechanischen Eigenschaften von Keratinsubstraten zu bewahren und/oder wiederherzustellen und/oder zu verbessern.

15. Verwendung nach Anspruch 12, **dadurch gekennzeichnet, dass** das modifizierte Polypeptid aus der KAP-Familie dazu dient, den Oberflächenzustand dieser Keratinsubstrate aufrechtzuerhalten und/oder wiederherzustellen und/oder zu verbessern.

16. Verwendung nach einem der Ansprüche 12 bis 15, **dadurch gekennzeichnet, dass** das modifizierte Polypeptid aus der KAP-Familie dazu dient, die Geschmeidigkeit von Keratinfasern und/oder deren Biegsamkeit und/oder deren Elastizität und/oder deren Bruchfestigkeit und/oder deren Volumen und/oder deren Frisur-Stabilität und/oder deren Widerstandsfähigkeit gegenüber chemischen Behandlungen zu verbessern.

17. Verwendung nach einem der Ansprüche 12 bis 16, **dadurch gekennzeichnet, dass** das modifizierte Polypeptid aus der KAP-Familie in der Zusammensetzung in einer Menge von 0,001 bis 30 Gew.-%, bezogen auf das Gesamtgewicht der Zusammensetzung, vorzugsweise von 0,01 bis 15 Gew.-% und noch stärker bevorzugt von 0,1 bis 5 Gew.-%, bezogen auf das Gesamtgewicht der Zusammensetzung, vorliegt.

18. Zusammensetzung, die in einem physiologisch verträglichen Medium, das an eine topische Applikation auf Keratinsubstrate angepasst ist, mindestens ein modifiziertes Polypeptid aus der KAP-Familie nach einem der Ansprüche 1 bis 6 umfasst.

19. Zusammensetzung nach Anspruch 18, **dadurch gekennzeichnet, dass** das modifizierte Polypeptid aus der KAP-Familie in der Zusammensetzung in einer Menge von 0,001 bis 30 Gew.-%, bezogen auf das Gesamtgewicht der Zusammensetzung, vorzugsweise von 0,01 bis 15 Gew.-% und noch stärker bevorzugt von 0,1 bis 5 Gew.-%, bezogen auf das Gesamtgewicht der Zusammensetzung, vorliegt.

20. Kosmetisches Verfahren, das Pflege, Behandlung, Stärkung und/oder Reparatur von Keratinsubstraten umfasst, **dadurch gekennzeichnet, dass** man auf die Haut, die Haare, die Wimpern oder die Nägel eine Zusammensetzung nach einem der Ansprüche 18 oder 19 aufbringt.

21. Verfahren nach Anspruch 20, **dadurch gekennzeichnet, dass** die Zusammensetzung auf sensibilisierte Keratinsubstrate, insbesondere sensibilisierte Keratinfasern, aufgebracht wird.

## Claims

1. Modified polypeptide of the KAP family, **characterized in that** it has the peptide sequence of access No. SwissProt Q9BYS0 or a fragment SPCCR thereof and **in that** it contains at least one salified amino acid and/or one amino acid modified by covalent bonding.

2. Modified polypeptide according to Claim 1, **characterized in that** the said fragment contains from 3 to 60 amino acids, in particular from 3 to 20 amino acids and even more preferentially from 3 to 10 amino acids.

3. Modified polypeptide according to either of Claims 1 and 2, **characterized in that** it contains from 1% to 100% of amino acids that are salified and/or modified by covalent bonding, preferably from 20% to 80% and even more preferentially from 30% to 60% of amino acids that are salified and/or modified by covalent bonding relative to the total number of amino acids that are salifiable and/or modifiable by covalent bonding of the said native polypeptide.

4. Modified polypeptide according to any one of Claims 1 to 3, **characterized in that** the said amino acids that are salifiable and/or modifiable by covalent bonding are chosen from the amino acids lysine, histidine, arginine, aspartate, glutamate, cysteine, methionine, tyrosine, threonine and serine.

5. Modified polypeptide according to any one of Claims 1 to 4, **characterized in that** it is a cationic polypeptide.

6. Modified polypeptide according to any one of Claims 1 to 4, **characterized in that** it is an anionic polypeptide.

7. Process for preparing a salified polypeptide of the KAP family, comprising the following steps:
- (a) a polypeptide of the KAP family having the peptide sequence of access No. SwissProt Q9BYS0 or a fragment SPCCR thereof and (b) at least one compound chosen from an acid and a base are reacted, in an aqueous, oily or emulsified base;
- the temperature is adjusted to between 20°C and 40°C and the pH to between 5 to 9; and
- the reaction is continued for a time ranging from 15 minutes to 3 hours.

8. Process according to Claim 7, **characterized in that** the acid is chosen from hydrochloric acid, acetic acid, succinic acid, palmitic acid, stearic acid, oleic acid, behenic acid, 18-methyleicosanoic acid, α-hydroxy acids and β-hydroxy acids, and mixtures thereof.

9. Process according to Claim 7, **characterized in that** the base is chosen from mineral bases, organic bases, guanidine derivatives, primary amines and tertiary amines, and mixtures thereof.

10. Process for preparing a polypeptide modified by covalent bonding, of the KAP family, via a substitution or acylation reaction comprising the following steps:
- (a) a polypeptide of the KAP family having the peptide sequence of access No. SwissProt Q9BYS0 or a fragment SPCCR thereof and (b) at least one carboxylic acid derivative are reacted, in an aqueous, oily or emulsified base or in a solvent-free medium;
- the temperature is adjusted to between 20°C and 180°C;
- the reaction is continued for a time ranging from 30 minutes to 8 hours.

11. Process for preparing a polypeptide modified by covalent bonding, of the KAP family, via a redox reaction comprising the following steps:
- (a) a polypeptide of the KAP family having the peptide sequence of access No. SwissProt Q9BYS0 or a fragment SPCCR thereof and (b) at least one thiol derivative are reacted, in an aqueous, oily or emulsified base or in a solvent-free medium;
- the temperature is adjusted to between 20°C and 80°C;
- the reaction is continued for a time ranging from 30 minutes to 8 hours.

12. Use of at least one modified polypeptide of the KAP family as defined in any one of Claims 1 to 6 in a cosmetic composition comprising a physiologically acceptable medium.

13. Use of at least one modified polypeptide of the KAP family as defined in any one of Claims 1 to 6 in a cosmetic composition, as an agent for maintaining and/or restoring and/or improving the protein content of the keratin substrate.

14. Use according to Claim 12, **characterized in that** the said modified polypeptide of the KAP family is intended for conserving and/or restoring and/or improving the physical and/or mechanical properties of keratin substrates.

15. Use according to Claim 12, **characterized in that** the said modified polypeptide of the KAP family is intended for maintaining and/or restoring and/or improving the surface state of the said keratin substrates.

16. Use according to any one of Claims 12 to 15, **characterized in that** the said modified polypeptide of the KAP family is intended for improving the softness of keratin fibres, and/or their suppleness and/or elasticity and/or resistance to breaking and/or volume and/or hairstyle hold and/or resistance to chemical treatments.

17. Use according to any one of Claims 12 to 16, **characterized in that** the said modified polypeptide of the KAP family is present in the composition in an amount ranging from 0.001% to 30% by weight relative to the total weight of the composition, preferably from 0.01% to 15% and even more preferentially from 0.1% to 5% by weight relative to the total weight of the composition.

18. Composition comprising, in a physiologically acceptable medium suitable for topical application to keratin substrates, at least one modified polypeptide of the KAP family as defined in any one of Claims 1 to 6.

19. Composition according to Claim 18, **characterized in that** the said modified polypeptide of the KAP family is present in the composition in an amount ranging from 0.001% to 30% by weight relative to the total weight of the composition, preferably from 0.01% to 15% and even more preferentially from 0.1% to 5% by weight relative to the total weight of the composition.

20. Cosmetic process including caring for, treating, strengthening and/or repairing keratin substrates, **characterized in that** a composition as defined in either of Claims 18 and 19 is applied to the skin, the hair, the eyelashes or the nails.

21. Process according to Claim 20, **characterized in that** the composition is applied to sensitized keratin substrates, in particular sensitized keratin fibres.
